(19) Europäisches Patentamt / European Patent Office / Office européen des brevets

(11) **EP 3 301 432 B1**

(12) **EUROPEAN PATENT SPECIFICATION**

(45) Date of publication and mention
of the grant of the patent:
**14.08.2024 Bulletin 2024/33**

(51) International Patent Classification (IPC):
**G01N 21/77** *(2006.01)* **G01N 21/27** *(2006.01)*
**G01N 33/493** *(2006.01)*

(21) Application number: **17194139.6**

(52) Cooperative Patent Classification (CPC):
**G01N 21/274; G01N 21/77; G01N 33/493;**
B01J 2219/00277

(22) Date of filing: **29.09.2017**

(54) **SPECIMEN ANALYSIS APPARATUS, AND MEASUREMENT METHOD THEREOF**

PROBENANALYSEVORRICHTUNG UND MESSVERFAHREN DAFÜR

APPAREIL D'ANALYSE D'ÉCHANTILLONS ET SON PROCÉDÉ DE MESURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2016 KR 20160125982**

(43) Date of publication of application:
**04.04.2018 Bulletin 2018/14**

(73) Proprietor: **Precision Biosensor Inc.
Daejeon 34036 (KR)**

(72) Inventors:
 • **Hwang, Kyu Youn**
 **Sejong-si (KR)**
 • **Shin, Sung Chul**
 **Gyeonggi-do (KR)**
 • **Park, Jong Myeon**
 **Gyeonggi-do (KR)**
 • **Lee, Sang Hyun**
 **Gyeonggi-do (KR)**
 • **Lee, Hae Seok**
 **Gyeonggi-do (KR)**

(74) Representative: **Germain Maureau et al
12, rue Boileau
69006 Lyon (FR)**

(56) References cited:
EP-A1- 2 618 161      EP-A1- 2 813 839
EP-A2- 0 453 036      WO-A1-2014/058750
DE-A1- 3 406 223      US-A- 5 462 879
US-A1- 2003 157 726      US-A1- 2013 203 613

**Description**

**BACKGROUND**

**1. Field**

Apparatuses and methods consistent with exemplary embodiments relate to a specimen analysis apparatus and method for measuring an optical signal value of a target material using the specimen analysis apparatus.

**2. Related Art**

A specimen analysis apparatus and method are required in various fields such as environmental monitoring, food inspection, medical diagnosis, etc. In the past, a skilled technician had to manually perform various processes, such as reagent injection, mixing, separation, migration, reaction, centrifugation, etc., multiple times to perform a test according to a predetermined protocol, but these manual processes could cause errors in the test results.

To reduce the problem, miniaturized and automated equipment to quickly analyze a test material has been developed. In particular, since a portable cartridge is able to quickly analyze a specimen anywhere, it could perform more various functions in more various fields if the structure and function of the portable cartridge is enhanced. Recently, studies on methods for precisely analyzing specimens with the miniaturized and automated specimen analysis apparatus are underway.

Patent document WO 2014/05 8750 A1 relates to a microfluidic testing cartridge for testing LAL-reactive substances in fluid samples. The cartridge may comprise at least two testing modules wherein each testing module includes at least one inlet port for receiving one of the fluid samples and at least four testing channels in fluid communication with the inlet port. Each of the testing channels may include a metering portion for metering an aliquot of the fluid sample, an analyzing portion: and a mixing portion, wherein a valve is positioned between the metering portion and the analyzing portion to selectively; fluidly separate the metering portion from the analyzing portion. The cartridge is insertable into an optical reader which performs optical measurements of the fluid sample within each testing channel during a testing process.

Patent document EP 0 453 036 A2 relates to a method of calibrating an analytical instrument, for example an atomic absorption spectrophotometer.

Patent document US 2003/ 157726 A1 relates to fluorescing reference material being used for calibration purposes for improving the measurement accuracy of sensors especially biosensors.

Patent document DE 34 06 223 A1 relates to a sample measuring device, a central processing unit for determining, by computation, a calibration curve on the basis of the measured values delivered by the sample measuring device and in accordance with the standard substance content of the samples.

Patent document US 2013/203613 A1 relates to a device and a method for the verification and quantitative analysis of analytes and their application for the verification and quantitative analysis of mycotoxins.

Patent document EP 2 813 839 A1 relates to a method of determine a physical property of a biological sample.

Patent document EP 2 618 161 A1 relates to a reagent accommodating/measuring cartridge for mixing and reacting a test specimen with a reagent and for measuring the reaction state thereof with the transmitted light, the cartridge comprising a plurality of transmitted-light measurement chambers having different optical path lengths.

Patent document US 5 462 879 A relates to a sensing instrument and method for measuring the concentration of an analyte.

**SUMMARY**

The presently claimed invention relates to a specimen analysis apparatus and a measurement method, as set out in claims 1 and 10, respectively.

Exemplary embodiments provide a specimen analysis apparatus and measurement method thereof, by which an optical signal value of a target material may be corrected by comparing an extent of change in optical signal value of the target material with a predetermined extent of change in optical signal.

According to an aspect of the present invention, there is provided a specimen analysis apparatus for determining a concentration of a target material in a specimen, comprising:

a cartridge, comprising at least two containers, each container containing a reagent configured to react with the target material in the specimen to measure the concentration of the target material, wherein each reagent further includes a predetermined concentration of internal standard material containing the target material, wherein the internal standard material is configured to release the target material while being dissolved during a process of mixing with the specimen and wherein the concentrations of the internal standard materials in the reagents of the at least two containers are different;

an optical detector unit for detecting optical characteristics from a reaction between the target material in the specimen and the released target material in the internal standard materials with the reagents in the at least two containers and providing optical signal values;

a controller configured to:

derive a measured calibration curve from an extent of a change in the optical signal value of the target material measured in the at least two containers;

obtain, from identification information provided on the cartridge and/or from information about the property of the reagent stored in a memory of the apparatus, a factory calibration curve indicating a predetermined extent of a change in the optical signal value of the target material in the at least two containers;

determine, based on a comparison of the measured calibration curve with the factory calibration curve, a correction value for calculation of the concentration of the target material; and

determine a corrected concentration of the target material based on the correction value.

A first container from among the at least two containers may not contain the internal standard material, and a second container from among the at least two containers may contain the internal standard material having a predetermined concentration.

The extent of the change in the optical signal values of the target material may include an extent of change in at least one of an absorbance, a fluorescence, and a luminance according to a difference of concentrations of the target material in the at least two containers.

The controller may be configured to determine a slope of a change in an absorbance based on a first absorbance of the target material measured in a first container from among the at least two containers, a second absorbance of the target material measured in a second container from among the at least two containers, and a difference between concentrations of the target material in the first and second containers, the correction value for the concentration of the target material being determined based on the slope of the change in the absorbance.

The controller may be configured to determine an amount of a change in an absorbance based on a first absorbance of the target material measured in a first container from among the at least two containers, a second absorbance of the target material measured in a second container from among the at least two containers, and a difference between concentrations of the target material in the first and second containers, the correction value for the concentration of the target material being determined using the calculated amount of change in absorbance based on the difference between concentrations of the target material in the first and second containers.

The controller may be configured to determine a slope of a change in a luminance based on a first luminance of the target material measured in a first container from among the at least two containers, a second luminance of the target material measured in a second container from among the at least two containers, and a difference between concentrations of the target material in the first and second containers, and the correction value for the concentration of the target material determine based the slope of the change in the luminance.

The controller may be configured to determine a slope of change in fluorescence based on a first fluorescence of the target material measured in a first container from among the at least two containers, a second fluorescence of the target material measured in a second container from among the at least two containers, and a difference between concentrations of the target material in the first and second containers, the correction value for the concentration of the target material being determined based one the slope of the change in the fluorescence.

The controller may be configured to determine at least one region between at least two specimen concentrations in the measured calibration curve corresponding to a difference in concentrations of the target material in the at least two containers and an extent of the change in the optical signal value of the target material for each region, and comparing the extent of the change in the optical signal value of the target material for each region with the factory calibration curve derived from the predetermined extent of the change in the optical signal value of the target material for the region to determine a correction value for a concentration of the target material for each region. The controller may be configured to generate a plot of concentration changes from an absorbance of the target material based on the correction value for the concentration of the target material. A predetermined extent of the change in the optical signal values of the target material may be stored in a memory or mapped to and stored in identification information provided on the cartridge. The controller may be configured to determine whether the optical signal values of the target material measured in the at least two containers are within a predetermined measurement range, and based on a result of the determination, determine whether to use the optical signal values in determining the correction value for the concentration of the target material.

According to an aspect of the present invention, there is provided a measurement method for determining a concentration of a target material in a specimen, the method comprising:

receiving the specimen in at least two containers, each container containing a reagent configured to react with the target material in the specimen to measure the concentration of the target material, wherein each reagent further includes a predetermined concentration of internal standard material containing the target material, wherein the internal standard material is configured to release the target material while being dissolved during a process of mixing with the specimen and wherein the concentrations of the internal standard materials in the reagents of the at least two containers are different;

detecting optical characteristics from a reaction between the target material in the specimen and the internal standard materials with the reagents in the at least two containers and providing optical signal values;

deriving a measured calibration curve from an extent of a change in the optical signal value of the target material measured in the at least two containers;

obtaining, from identification information provided on the cartridge and/or from information about the property of the reagent stored in a memory of the apparatus, a factory calibration curve indicating a predetermined extent of a change in the optical signal value of the target material in the at least two containers;

determining, based on a comparison of the measured calibration curve with the factory calibration curve, a correction value for calculation of the concentration of the target material; and

determining a corrected concentration of the target material based on the correction value.

The determining the correction value for the concentration of the target material may include: determining a slope of change in an absorbance based on a first absorbance of the target material measured in a first container from among the at least two containers, a second absorbance of the target material measured in a second container from among the at least two containers, and a difference between concentrations of the target material in the first and second containers; the correction value for the concentration of the target material being determined based on the slope of change in absorbance.

The determining the correction value for the concentration of the target material may include: determining an amount of change in an absorbance based on a first absorbance of the target material measured in a first container from among the at least two containers, a second absorbance of the target material measured in a second container from among the at least two containers, and a difference between concentrations of the target material in the first and second containers; the correction value for the concentration of the target material being determined by comparing the amount of the change in the absorbance with a predetermined amount of the change in the absorbance.

The determining the correction value for the concentration of the target material may include: determining a slope of a change in a luminance based on a first luminance of the target material measured in a first container from among the at least two containers, a second luminance of the target material measured in a second container from among the at least two containers, and a difference between concentrations of the target material in the first and second containers; the correction value for the concentration of the target material being determined based on the slope of change in the luminance.

The determining the correction value for the concentration of the target material may include: determining a slope of a change in a fluorescence based on a first fluorescence of the target material measured in a first container from among the at least two containers, a second fluorescence of the target material measured in a, and a difference between concentrations of the target material in the first and second containers; the correction value for the concentration of the target material being determined based on the slope of change in the fluorescence.

The measurement method may include: determining at least one region corresponding to a difference between concentrations of the target material in the at least two containers and an extent of a change in an optical signal value of the target material for each region, and comparing the extent of the change in the optical signal value of the target material for each region with a predetermined extent of the change in the optical signal value of the target material for the region to determine the correction value for the concentration of the target material for each region; generating a plot of concentration changes according to an absorbance of the target material based on the correction value for the concentration of the target material; and determining whether the optical signal values of the target material measured in the at least two containers are within a predetermined measurement range, and based on a determination result, determining whether to use the optical signal values in determining the correction value for the concentration of the target material.

BRIEF DESCRIPTION OF THE DRAWINGS

While the invention is defined in the appended claims, the above and/or other aspects will become more apparent to those of ordinary skill in the art by describing in detail examples with reference to the accompanying drawings, in which:

FIG. 1 shows a specimen analysis apparatus combined with a cartridge,

FIG. 2 shows a cartridge and an install member of a specimen analysis apparatus to be combined with the cartridge, which are separated from each other,

FIG. 3 shows a cartridge and an install member of a specimen analysis apparatus, which are combined together,

FIG. 4 shows a cartridge,

FIG. 5 is an exploded view of a test unit of a cartridge,

FIG. 6 shows a cartridge with a plurality of containers containing reagents that have an internal standard material with different concentrations,

FIG. 7 is a cross-sectional view of a test unit of the cartridge of FIG. 4 cut along AA'.

FIG. 8 is a block diagram of a specimen analysis apparatus,

FIG. 9 is a diagram for explaining detection of optical characteristics of a target material provided in a container,

FIG. 10A shows how to set an optical signal value of a target material in a coordinate system, FIG. 10B shows how to create a plot based on coordinates set based on optical signal values of a target material,

FIGS. 11A and 11B are graphs representing changes in concentration of a target material,

FIG. 12 shows determination of a correction value by sectionalizing a plot,

FIG. 13 shows determination of a correction value by sectionalizing a plot of change in concentration of a target material,

FIGS. 14A and 14B show a predetermined measurement

FIG. 15 shows a user interface screen displayed on a display, which is configured to provide a corrected concentration of a target material,

FIG. 16 shows a user interface screen displayed on a display, which is configured to provide a corrected concentration of a target material and a plot of the corrected concentration of the target material,

FIG. 17 is a flowchart illustrating operation of a specimen analysis apparatus for determining a correction value for a concentration of a target material, and

FIG. 18 is a flowchart illustrating operation of a specimen analysis apparatus for determining a correction value by selecting an optical signal value from within a normal measurement range.

## DETAILED DESCRIPTION OF EXEMPLARY EMBODIMENTS

The terminology used herein is for the purpose of describing particular examples only and is not intended to limit the present disclosure. It is to be understood that the singular forms "a," "an," and "the" include plural references unless the context clearly dictates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The terms including ordinal numbers like "first" and "second" may be used to explain various components, but the components are not limited by the terms. The terms are only for the purpose of distinguishing a component from another. For example, the first component may be termed as the second component, and vice versa, within the scope of the present invention. Descriptions shall be understood as to include any and all combinations of one or more of the associated listed items when the items are described by using the conjunctive term "~ and/or ~," or the like.

Furthermore, the terms as used throughout the specification, such as "- part", "- block", "~ member", "- module", etc., may mean a unit of handling at least one function or operation. For example, it may refer to software or hardware, such as field programmable gate arrays (FPGAs) or application specific integrated circuits (ASICs). However, the terms "- part", "- block", "~ member", "- module", etc., are not limited to software or hardware, but may be any element to be stored in an accessible storage medium and processed by one or more processors.

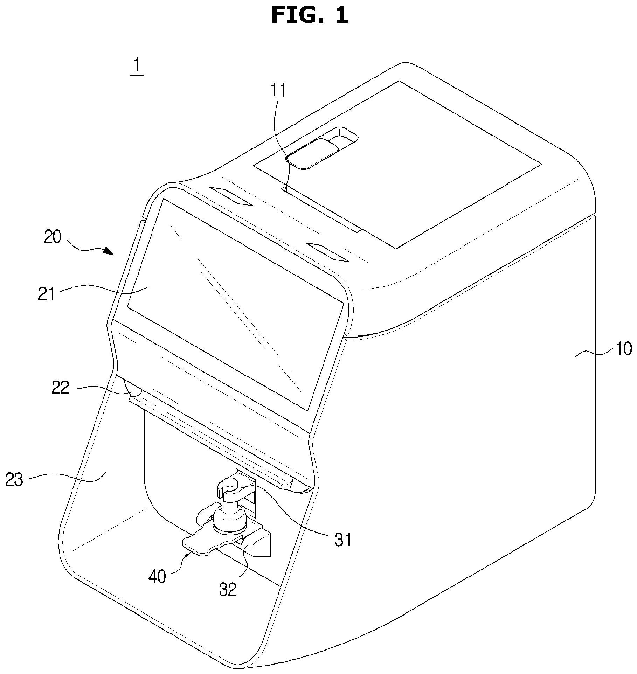

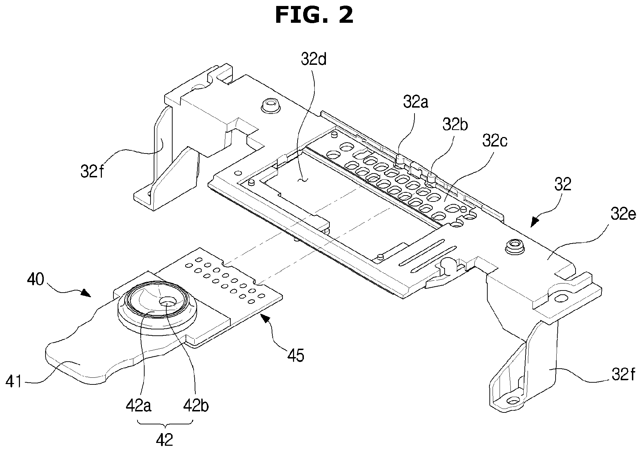

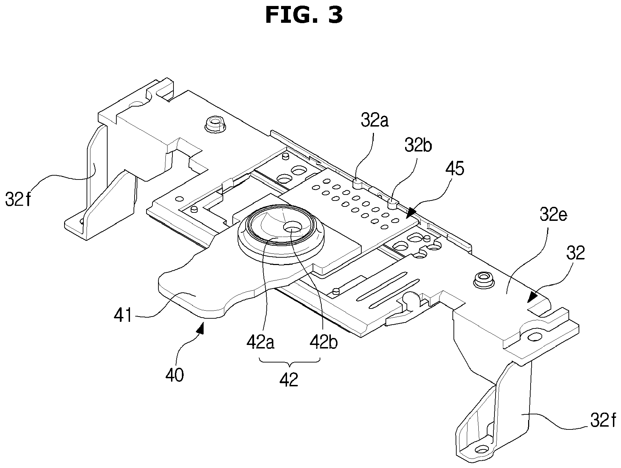

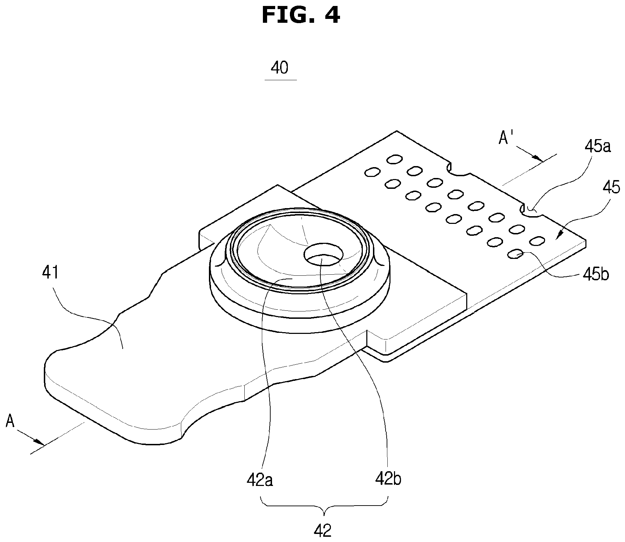

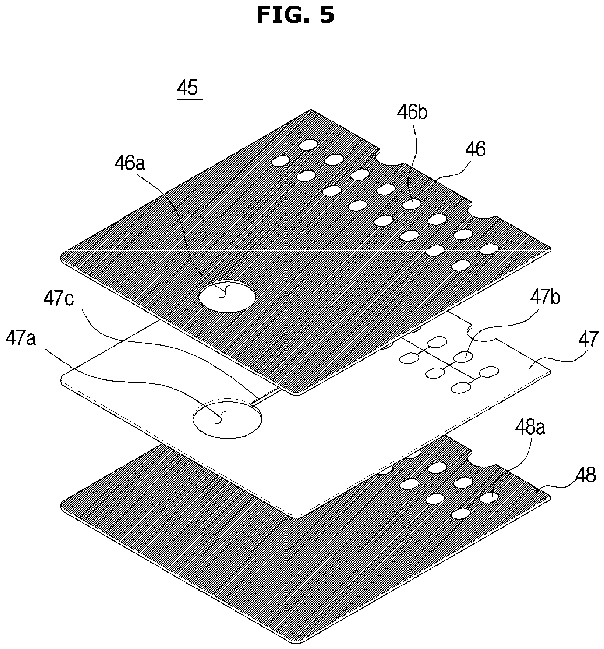

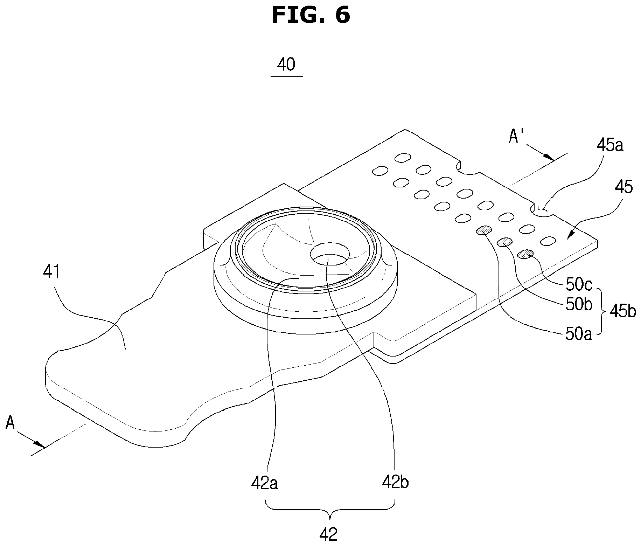

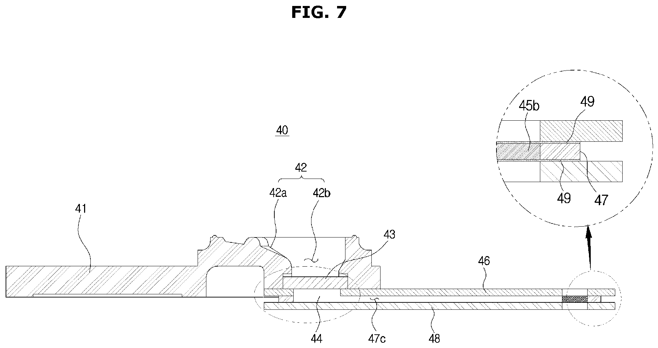

FIG. 1 shows a specimen analysis apparatus combined with a cartridge, FIG. 2 shows a cartridge and an install member of a specimen analysis apparatus to be combined with the cartridge, which are separated from each other, and FIG. 3 shows a cartridge and an install member of a specimen analysis apparatus, which are combined together. FIG. 4 shows a cartridge, and FIG. 5 is an exploded view of a test unit of a cartridge. FIG. 6 shows a cartridge with a plurality of containers containing reagents that have an internal standard material with different concentrations, and FIG. 7 is a cross-sectional view of a test unit of the cartridge of FIG. 4 cut along AA'. FIGS. 1 to 7 will be collectively described to avoid overlapping explanation. Referring to FIG. 1, a specimen analysis apparatus 1 combined with a cartridge 40 includes a housing 10 that forms the exterior, and a door module 20 installed on the front of the housing 10. The door module 20 may include a display 21, a door 22, and a door frame 23. The display 21 and the door 22 may be arranged on the front of the door frame 23. As shown in FIG. 1, the display 21 may be placed above the door 22, but it may also be located anywhere that may provide various visual information for the user.

The door 22 may be slidingly installed, and configured to be positioned behind the display 21 when slid open.

The display 21 may display various specimen analysis information, such as the concentration of a target material in the specimen. If the display 21 is implemented as a touch screen type, the display 21 may receive various information and commands from the user.

For example, the display 21 may display icons for input of various control commands related to the specimen analysis apparatus 1. The display 21 may display a user interface configured to provide not only the icons related to controlling the specimen analysis apparatus 1 but also analysis result information. This will be described in detail later.

The door frame 23 may be equipped with an install member 32 on which the cartridge that contains various reagents may be mounted. The user may slide the door 22 open upward to mount the cartridge 40 on the install member 32, and slide the door 22 closed downward to perform analysis operation.

A specimen is injected to the cartridge 40 and reacts with a reagent in a test unit 45. The cartridge 40 is inserted into the install member 32, and a pressurizing member 31 presses the cartridge 40 for the specimen in the cartridge 40 to flow into the test unit 45.

Furthermore, apart from the display 21, the specimen analysis apparatus 1 may further include an output unit 11 for outputting the analysis result in printed form. Accordingly, the user may check the analysis result through the output unit 11 as well as through the display 21.

Referring to FIGS. 2 to 4, the cartridge 40 may be inserted into the install member 32 of the specimen analysis apparatus 1. The install member 32 may include a seat 32c for the cartridge 40 to be seated therein, and supporters 32f for supporting the install member 32 in the specimen analysis apparatus 1.

The supporters 32f may be formed to extend from either ends of a body 32e of the install member 32, and the seat 32c may be arranged in the central part of the body 32e. A slit 32d may be formed behind the seat 32c to prevent an error that might occur in measuring a test result of a specimen sample in the test unit 45.

The install member 32 may include contacts 32a, 32b for contacting the cartridge 40, and the test unit 45 of the cartridge 40 may include depressions 45a having the form corresponding to the contacts 32a, 32b. Thus, the depressions 45a and the contacts 32a, 32b come into contact with each other. There may be two depressions 45a and two corresponding contacts 32a, 32b, without being limited thereto.

The cartridge 40 may include a housing 41 that forms the exterior and the test unit 45 in which the specimen and the reagent are joined and make a reaction.

The housing 41 may be implemented in a form that supports the cartridge 40 and allows the user to be able to grip the cartridge 40. The housing 41 may have a user-gripping part implemented in the form of a streamlined protrusion, as shown in FIGS. 2 and 3. Accordingly, the user may grip the cartridge 40 stably. However, the housing 41 is not limited to the form shown in the drawings, and may be implemented in various shapes.

Furthermore, a specimen supplier 42 may be arranged in the cartridge 40 to receive a specimen. The specimen supplier 42 may include a supply hole 42b through which a specimen sample flows into the test unit 45, and an auxiliary supplier 42a for assisting in supply of the specimen.

A specimen to be tested in the specimen analysis apparatus 1 may be supplied to the specimen supplier 42. The specimen herein used may also be referred to as a sample, including a bio sample such as blood, tissue fluid, lymph fluid, saliva, and urine, or an environmental sample for water-purity control or soil conservation, without being limited thereto. For the specimen, a diluted or non-diluted sample may be used, without being limited thereto.

The supply hole 42b may have a circular form as shown in FIG. 2, but is not limited thereto. For example, the supply hole 42b may have a polygonal form. The user may use a tool such as a pipette or a syringe to drop the specimen onto the specimen supplier 42.

The auxiliary supplier 42a may be formed around the supply hole 43b to be slanted toward the supply hole 42b, enabling the specimen dropped around the supply hole 42b to flow down to the supply hole 42b. Specifically, if the user fails to drop the specimen directly into the supply hole 42b but drops it around the supply hole 42b, the specimen may flow into the supply hole 42b along the slope of the auxiliary supplier 42a.

Furthermore, in addition to facilitating supply of a specimen, the auxiliary supplier 42a may also prevent contamination of the cartridge 40 due to a wrongly-supplied specimen. Specifically, even if the specimen fails to go directly into the supply hole 42b, the auxiliary supplier 42a around the supply hole 42b may prevent the specimen from flowing to the test unit 45 or the gripping part, thereby preventing contamination of the cartridge 40 from the specimen and preventing the specimen that might be harmful to the human body from contacting the user.

Although the drawings show that the specimen supplier 42 includes a single supply hole 42b, the present disclosure is not limited thereto and a plurality of supply holes 42b may be provided. In the latter case, the single cartridge 40 may perform tests on many different specimens simultaneously. The different specimens may be the same type but come from different sources, or may be different types and come from different sources, or may be the same type and come from the same source but may be put in different states.

As described above, since it is often the case that the housing 41 comes into contact with a specimen while having a form that facilitates a particular function, the housing 41 may be formed of an easy-to-mold and chemically and biologically inactive material.

For example, the housing 41 may be made of any of various materials including plastic materials, such as acryl, e.g., polymethylmethacrylate (PMMA), polysiloxane, e.g., polydimethylsiloxane (PDMS), polycarbonate (PC), linear low density polyethylene (LLDPE), low density polyethylene (LDPE), medium density polyethylene (MDPE), high density polyethylene (HDPE), polyvinyl alcohol, very low density polyethylene (VLDPE), polypropylene (PP), acrylonitrile butadiene styrene (ABS), cycloolefin copolymer (COC), etc., glass, mica, silica, semiconductor wafer, etc.

The aforementioned materials are, however, merely examples of materials to be used for the housing 41, without being limited thereto. The housing 41 may be implemented with any material having chemical and biological stability and mechanical processability.

The cartridge 40 may be configured to be combined or joined with the test unit 45. A specimen injected through the specimen supplier 42 may flow into the test unit 45 and may be tested as it makes a reaction with a reagent. The test unit 45 may include a plurality of containers 45b, each containing a reagent to react with the specimen.

In this regard, various kinds of reagents may be provided in the plurality of containers 45b. For example, a reagent used to detect the concentration of a target material in the specimen may be provided in the container 45b. For example, the reagent may include a coupler that shows different optical characteristics depending on concentrations of the target material in the specimen, so the specimen analysis apparatus 1 may detect an optical characteristic through a photo detector and calculate a concentration of the target material based on the detection result. This will be described in detail later.

Hereinafter, the target material refers to a material to be analyzed in the specimen. For example, the target material may be protein, enzyme, or ion included in the specimen, without being limited thereto. If the specimen is human blood, the target material may be one of various materials that exist in the blood, such as sodium (Na), potassium (K), etc., without being limited thereto. This will be described in detail later, and in the following description, a structure of the test unit 45 of the cartridge 40 will be described in detail.

Referring to FIG. 5, the test unit 45 of the cartridge 40 may have a structure in which three plates 46, 47, 48 are joined. The three plates 46, 47, 48 may be divided into a top plate 46, a middle plate 47, and a bottom plate 48. The top and bottom plates 46 and 48 may be printed with light shielding ink to protect the specimen sample moving into the container 45b against light or prevent an error that might occur in measuring the optical characteristic in the container 45b. The top and bottom plates 46 and 48 may each be about 10 to 300 μm thick. The middle plate 47 may have the thickness of about 50 to 300 μm.

The top and bottom plates 46 and 48 of the test unit 45 may be formed of a film selected from among polyethylene films, such as VLDPE, LLDPE, LDPE, MDPE, HDPE, etc., PP films, PVC films, PVA films, polystyrene (PS) films, and PET films. The films are merely examples, and there are no limitations on the films used to form the top and bottom plates 46 and 48 as long as the films are chemically and biologically inactive and mechanically processable.

Unlike the top and bottom plates 46 and 48, the middle plate 47 of the test unit 45 may be formed of a porous sheet. For example, the porous sheet to be used for the middle plate 47 may include one or more of cellulose acetate, nylons (e.g., nylon 6.6, nylon 6.10), and polyether sulfone. Since the middle plate 47 is formed of a porous sheet, it serves as a vent by itself, allowing the specimen to move inside the test unit 45 without the need for an extra driving source. Furthermore, if the specimen has a hydrophile property, the middle plate 47 may be coated with a hydrophobic solution to prevent the specimen sample from permeating the middle plate 47.

An inlet 46a, through which a specimen flows in, may be formed in the top plate 46, and an area 46b corresponding to the containers 45b may be transparent. In the bottom plate 48, an area 48a corresponding to the containers 45b may also be transparent. This is to measure the absorbance, an example of optical characteristic from a reaction that occurs in the containers 45b. The absorbance is also referred to as reflection or transmission density, but for convenience of explanation, the former term will be used herein.

The middle plate 47 may also have an inlet 47a for a specimen to flow in therethrough, and the inlet 46a of the top plate 46 and the inlet 47a of the middle plate 47 overlap to form an inlet 44 of the test unit 45 (see FIG. 7). Furthermore, the middle plate 47 may have an area 47b corresponding to the containers 45b and a fluid path 47c to connect the inlet 47a and the container 45b.

Many different reactions may occur in the test unit 45 for analysis of the specimen. In a case of using blood for the specimen, a reagent that comes in a color or changes colors according to a reaction with a particular ingredient of the blood (blood plasma in particular) may be provided in the container 45b to optically detect and evaluate the color appearing in the container 45b. Based on the evaluations, whether there exists the particular ingredient in the blood, a ratio of the particular ingredient, the concentration, or the like may be determined.

Referring to FIGS. 6 and 7, the cartridge 40 may be formed in such a manner that the test unit 45 is joined to the bottom of the housing 41. Specifically, the test unit 45 may be joined to the bottom of the specimen supplier 42 through the supply hole 42b formed thereon.

A pressure sensitive adhesive (PSA) may be used to join the housing and the test unit 45 together, and the PSA has properties of being adhered to an object in short period of time with low pressure, such as finger pressure, at a room temperature, preventing cohesive failure, and leaving no residuum on the surface of the object. However, the housing 41 and the test unit 45 are joined not only by the PSA but may also be joined by a double-sided adhesive or in a method of being inserted into a groove.

The specimen flowing in through the supply hole 42b passes a filtering unit 43 and then flows into the test unit 45. The filtering unit 43 may be put into the supply hole 42b of the housing 41. The filtering unit 43 may include at least one porous membrane with many pores to filter materials included in the specimen sample, which are bigger than a predetermined size. For example, the filtering unit 43 may include two-layered filters, i.e., a first or upper filter and a second or lower filter. The first filter may be formed of glass fiber, non-woven fabric, absorbent filter, etc., and the second filter may be formed of PC, PES, PE, polyacrylic sulfone (PASF), etc.

With the two-layered filtering unit 43, the second filter may filter the specimen again, which has already passed through the first filter. Furthermore, it may protect the filtering unit 43 from being torn or damaged in case that a lot of particles of a size greater than the pore of the filtering unit 43 flow in. The filtering unit 43 is not, however, limited thereto, and may be implemented in a structure of three or more layers, or a single layer. In the case of three or more layers, filtering performance of the filtering unit 43 on the specimen may be further enhanced and the stability may be improved as well. The filtering unit 43 may be processed with adhesive materials such as double-sided adhesives.

The test unit 45 may include an inlet 44 through which the specimen that has passed the filtering unit 43 flows in, the fluid path 47c along which the specimen flowing in is moved, and the containers 45b in which a reaction occurs between the specimen and a reagent.

The top, middle, and bottom plates 46, 47, and 48 may be joined together by double-sided adhesives. Specifically, a double-sided tape 49 is attached to the top and bottom faces of the middle plate 47, making the top, middle, and bottom plates 46, 47, and 48 combined together. The containers 45b of the cartridge 40 may be separated from another. For example, as shown in FIG. 7, the containers 45b may include a first container 50a, a second container 50b, and a third container 50c, but the number of the containers is not limited thereto. In the following description, for convenience of explanation, the first and second containers may be called sub-containers if there is no need to distinguish one from another, and the sub-containers are collectively called a container.

The first, second, and third containers 50a, 50b, and 50c may contain different reagents to react with a target material with different concentrations of internal standard material. The specimen analysis apparatus 1 may produce various results by analyzing the specimen flowing into the container 45b using an optical detector 100. The respective elements of the specimen analysis apparatus 1 will now be examined.

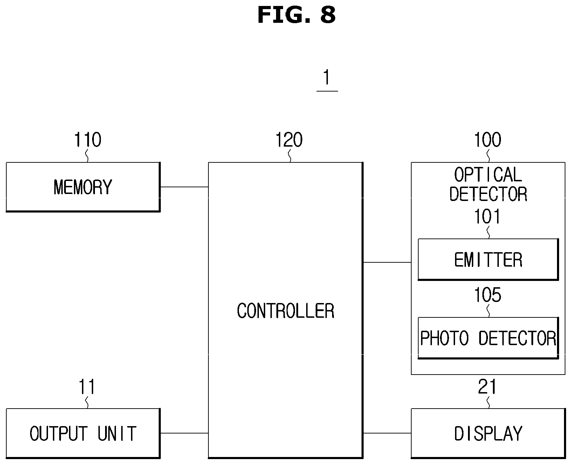

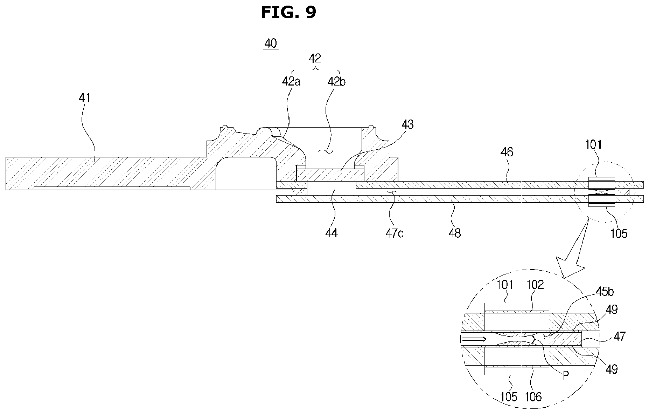

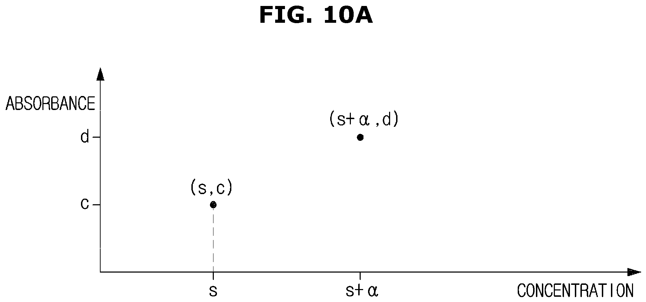

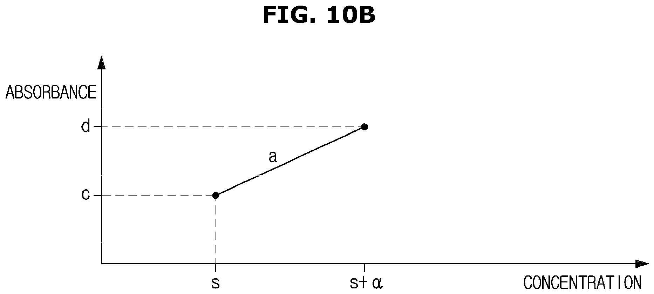

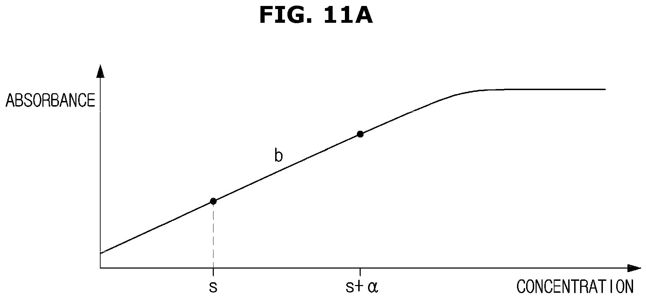

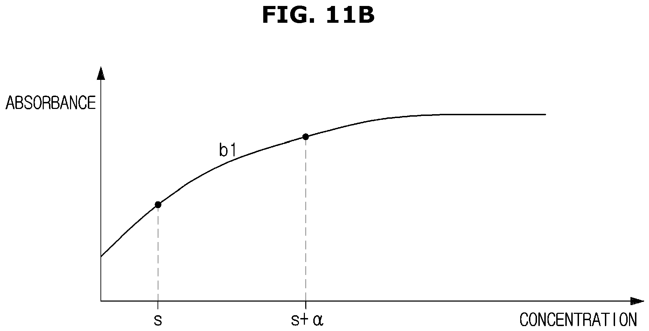

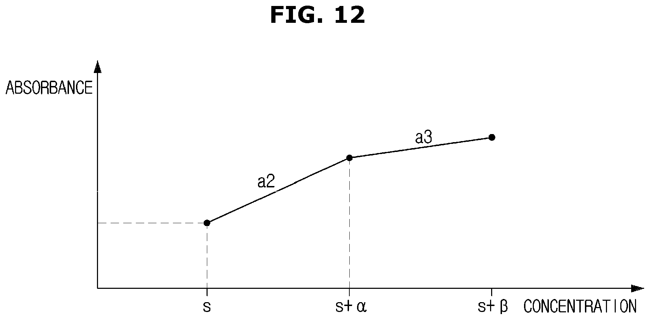

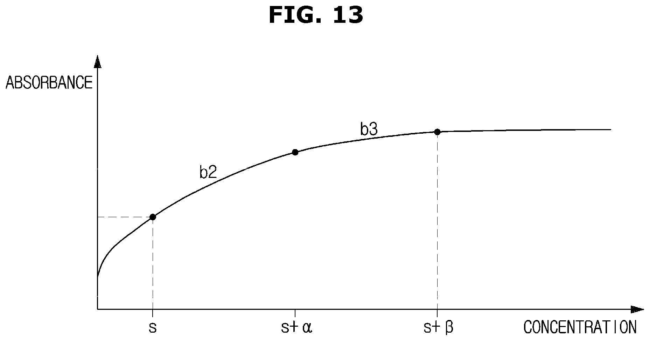

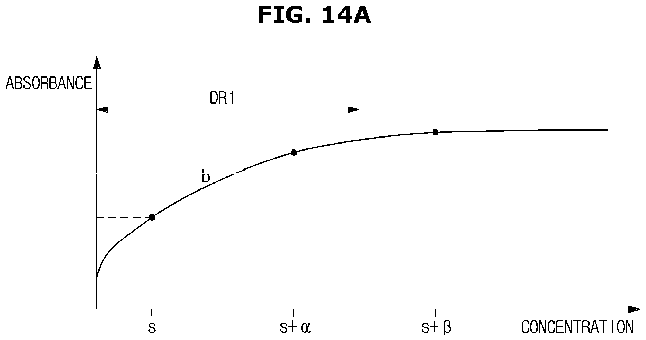

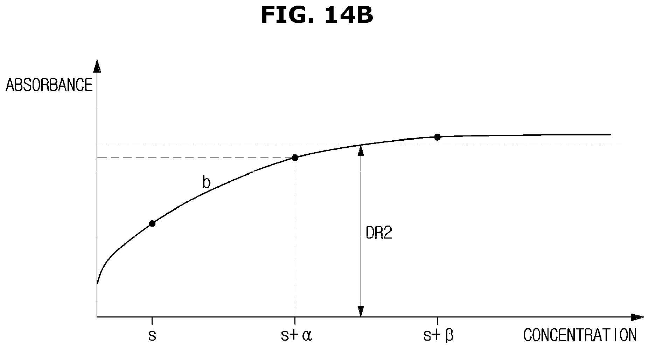

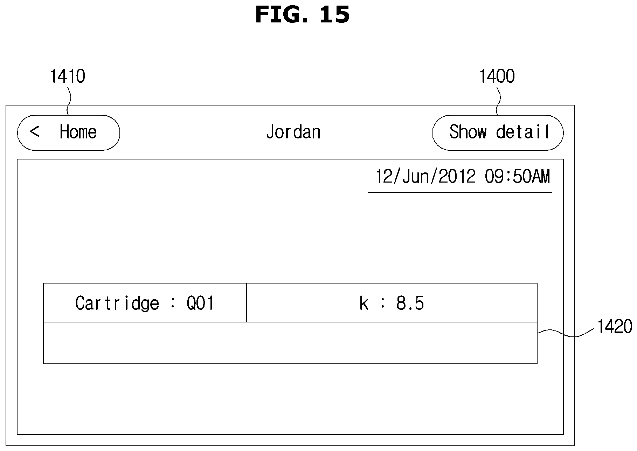

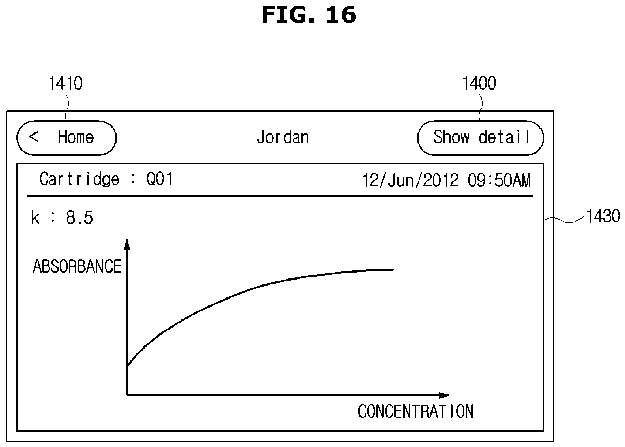

FIG. 8 is a block diagram of a specimen analysis apparatus, and FIG. 9 is a diagram for explaining detection of optical characteristics of a target material provided in a container. FIG. 10A shows how to set an optical signal value of a target material in a coordinate system, FIG. 10B shows how to generate a plot based on coordinates set based on optical signal values of a target material. FIGS. 11A and 11B are graphs representing changes in concentration of a target material, FIG. 12 shows determination of a correction value by sectionalizing a plot, FIG. 13 shows determination of a correction value by sectionalizing a plot of change in concentration of a target material, and FIGS. 14A and 14B show a predetermined measurement range. FIG. 15 shows a user interface screen displayed on a display, which is configured to provide a corrected concentration of a target material, and FIG. 16 shows a user interface screen displayed on a display, which is configured to provide a corrected concentration of a target material and a plot of the corrected concentration of the target material. FIGS. 8 to 16 will be collectively described to avoid overlapping explanation.

Referring to FIG. 8, the specimen analysis apparatus 1 may include an optical detector 100 for detecting optical characteristics from a reaction between a specimen and a reagent provided in the container 45b, a memory 110 for storing control data, programs, or the like required to control overall operation of the specimen analysis apparatus 1, a controller 120 for controlling general operation of the specimen analysis apparatus 1, an output unit 11 for outputting a result of analyzing the specimen, and a display 21 for visually providing various information. The output unit 11 and the display 21 were described above, so the description thereof will be omitted.

The optical detector 100 may detect optical characteristics, and the controller 120 may calculate many different analysis values to be used in diagnosis of a target subject, such as concentrations of a target material based on the detection results, as will be described below.

The optical detector 100 may include an emitter 101 for emitting light, and a photo detector 105 for detecting light. The emitter 101 may be implemented with various types of well-known sensors and devices that emit light, such as a light emitting diode (LED) sensor, an infrared sensor, or the like, without being limited thereto.

The photo detector 105 may collect or detect light in the container 45b. For example, the photo detector 105 may be implemented by a photo diode that detects light in the container 45b and converts the light into electric energy, but is not limited thereto.

The emitter 101 and the photo detector 105 may be arranged to face each other with the container 45b centered between them. Accordingly, the photo detector 105 may detect an optical characteristic from a reaction between a reagent and a specimen in the container 45b.

For example, a reagent P may be provided in the container 45b in advance. Referring to FIG 9, the reagent P is in a dried solid state and applied on one or more sides of the container 45b. The emitter 101 may be located adjacent to an area 46b formed in the top plate 46 to emit light to the container 45b, as shown in FIG. 9. In this regard, to prevent contamination of the emitter 101 from the reagent, specimen, etc., a transparent plate 102 may be attached to a side of the emitter 101, without being limited thereto.

Correspondingly, the photo detector 105 may be arranged adjacent to an area 48a formed in the bottom plate 48 to detect or collect light emitted by the emitter 101 to the container 45b. A transparent plate 106 may also be attached to a side of the photo detector 105 to prevent contamination thereof from the reagent, specimen, etc., without being limited thereto.

Positions of the emitter 101 and photo detector 105 are not limited to what are shown in the drawings. For example, the emitter 101 may be arranged at a location adjacent to the area 48a formed in the bottom plate 48 and the photo detector 105 may be arranged at a location adjacent to the area 46b formed in the top plate 46.

In a case that the photo detector 105 is implemented with a photo diode, the magnitude of electric energy output from the photo detector 105 may vary depending on the concentration of the target material. The controller 120 may calculate the absorbance, concentration, etc., based on the electric energy output from the photo detector 105.

A reagent may be provided in advance to at least one sub-container, as described above. The reagent is used to measure the concentration of a target material and provided in the container 45b in a dried solid state. As a specimen is injected along the fluid path 47c, the reagent contacts the specimen, mingling with the specimen while changing into a liquid.

The reagent may include various materials to measure a target material present in the specimen. For example, the reagent may include a coupler having different optical characteristics depending on the concentration of the target material in the specimen.

In another example, the reagent may include a material to keep the PH at the proper level in order to detect an optical characteristic of the specimen more correctly. If the PH of the specimen is out of a particular range, it makes it difficult to measure the absorbance, so the reagent may include the material to buffer the PH of the specimen.

In another example, the reagent may include a material having an ion-selective property that attracts a particular ion. By attracting the target material using the material, the concentration of the target material may be measured more correctly.

Furthermore, the reagent may include an internal standard material. The internal standard material is a material that contains the target material and releases the target material while being dissolved during a process of mixing with the specimen.

For example, if, among the materials present in the specimen, sodium (Na) corresponds to the target material, sodium chloride (NaCl) may be selected as the internal standard material. Once the specimen flows into the container 45b, the internal standard material may be dissolved into ions Na+, Cl- while changing from solid to liquid as it is mixed with the specimen. The internal standard material may change the concentration of the target material in the container 45b as it is dissolved.

The containers may contain the same or different reagents. Accordingly, the user may easily obtain the concentration of the target material to be measured by selecting a cartridge 40 containing a reagent related to the target material and installing the cartridge 40 in the frame 32. Concentrations of the internal standard material included in the reagents provided in the respective sub-containers may be set equally or differently. For example, at least one of the sub-containers may contain a reagent that does not include the internal standard material, i.e., zero concentration of internal standard material. A reagent including a predetermined concentration of internal standard material may be injected to at least another one of the sub-containers.

Accordingly, if the specimen is injected to the sub-containers, concentration, absorbance, luminance, and fluorescence of the measured target material may be the same or different for each sub-container. Hereinafter, values that may be derived using optical characteristics of the specimen such as concentration, absorbance, fluorescence, luminance of the target material are collectively called optical signal vales of the target material, and the same is true in the case that there is no need to distinguish the concentration, absorbance, fluorescence, luminance of the target material.

Concentrations of the internal standard material injected to the respective sub-containers may be set in advance. For example, the cartridge 40 may be manufactured with predetermined reagents provided in the respective sub-containers, and information about the injected reagents may be mapped to the identification information attached to the cartridge 40. The identification information is provided to identify the cartridge 40, such as QR code, bar code, etc., and may be mapped and stored with information about a property of the reagent injected to the container 45b. The information about the property of the reagent may include information about an extent of change in concentration of the target material according to an extent of change in optical signal value of the target material. Furthermore, the information about the property of the reagent may include information regarding the concentration of the injected internal standard material and the concentration of the target material that comes out as a reaction is made with the specimen.

For example, the information about the property of the reagent may include a calibration curve derived based on measurements in an ideal state. The calibration curve stored in the information about the property of the reagent may be called a factory calibration curve. As will be described later, the controller 120 may determine a correction value by comparing the factory calibration curve and the calibration curve derived from the container 45b.

The specimen analysis apparatus 1 is equipped with an image sensor to recognize the identification information and determine the information about the property of the reagent. If the display 21 is implemented as a touch screen type, the user may input a bar code number to the display 21 and the specimen analysis apparatus 1 may obtain the information about the property of the reagent. The information about the property of the reagent may be stored in the memory 110 of the specimen analysis apparatus 1, without being limited thereto.

The controller 120 may determine a difference in concentration of the target material between the sub-containers from the identification information, and calculate an extent of change in the optical signal value of the target material between the sub-containers from a detection result of the optical detector 100.

The extent of change in the optical signal value of the target material between the sub-containers may include at least one of an extent of change in absorbance, an extent of change in fluorescence, and an extent of change in luminance of the target material between the sub-containers.

Accordingly, the controller 120 may calculate the extent of change in the optical signal value based on the difference in concentration of the target material, and determine a correction value for a concentration of the target material by comparing the difference in concentration of the target material mapped to the identification information with the extent of change in the optical signal value. The controller 120 will be described in more detail later.

As described above, the reagent includes a chromophore that shows different optical characteristics depending on the amount of a particular ingredient in the specimen, but the chromophore may change in property due to light, oxygen, moisture, etc., and thus has a problem that the measurement accuracy may decrease as time passes. Furthermore, with the lapse of time, it suffers from not only a change of the reagent itself but also a decrease in measurement accuracy if there is an interfering substance that interferes with a reaction between the reagent and the target material in the container.

The specimen analysis apparatus 1 may compensate for or correct an optical signal value of the target material by determining a correction value for the optical signal value based on comparison of an extent of change in optical signal value between at least two containers with a predetermined extent of change, e.g., a calibration curve mapped to the identification information and derived in a normal state.

Accordingly, the specimen analysis apparatus 1 may increase the term of validity of the cartridge, i.e., an effective life of the reagent, and diagnose a subject more correctly. This will be described in detail later, and a control block diagram of the specimen analysis apparatus 1 will now be described.

Referring to FIG. 8, the memory 110 of the specimen analysis apparatus 1 may be implemented in at least one of flash memory type, hard disk type, multimedia card micro type, card type (e.g., SD or XD memory), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, and optical disk. The memory 110 is not, however, limited thereto, and may be implemented in any type well-known in the art.

The memory 110 may store control data, control programs, etc., used to control general operation of the specimen analysis apparatus 1. Accordingly, the controller 120 may control general operation of the specimen analysis apparatus 1 using the data stored in the memory 110.

The memory 110 may also store instructions for executing a method for correcting the optical signal value of a target material. For example, the instructions may be stored in the memory 110 in the form of a program. Accordingly, the controller 120 may perform a correction process for the optical signal value using the data stored in the memory 110, as will be described below.

The memory 110 may also store data related to the information about the property of the reagent. The data related to the information about the property of the reagent is not only stored in the memory 110 but also mapped to and stored in the identification information provided on the cartridge 40 as described above.

In the case that the information about the property of the reagent is mapped to the identification information, the capacity of the memory 120 may be more efficiently managed. Furthermore, if the information about the property of the reagent needs to be updated or replaced, the update or replacement may be easily performed because only the identification information needs to be replaced without the need to update the memory 120.

The memory 110 may also store data about a user interface. The user interface refers to an environment configured to allow the user to easily input various setting instructions or control command for the specimen analysis apparatus 1, easily control the program stored in the memory 110, and easily obtain various information such as analysis results from the specimen analysis apparatus 1.

For example, the user interface may be a graphic user interface (GUI) that graphically implements screens to be displayed on the display 21 in order to more conveniently perform exchange of various information between the user and the specimen analysis apparatus 1.

Methods for providing various information and displaying and arranging icons to receive various setting instructions and control commands may be implemented in an algorithm or program and stored in the memory 110. Accordingly, the controller 120 may generate a user interface using the data stored in the memory 110 and display the user interface on the display 21. Alternatively, the algorithm or program may be stored in an external device. In this case, the controller 120 may receive data related to the user interface derived from the algorithm or program over a communication network and display the user interface on the display 21, without being limited thereto.

The data stored in the memory 110 may be updated. For example, the user interface, the method for compensating for the optical signal value of the target material, or the data related to the information about the property of the reagent stored in the memory 110 may be updated over a wired or wireless communication network, without being limited thereto. In this regard, the data stored in the memory 110 may be updated according to a control command of the user or automatically updated at predetermined intervals, without being limited thereto.

The memory 110 and the controller 120 may be implemented separately or integrated in a system on chip (SOC) embedded in the specimen analysis apparatus 1, without being limited thereto. The controller 120 will now be described in detail.

The controller 120 may be equipped in the specimen analysis apparatus 1. The controller 120 may be implemented by a device that is capable of processing various operations, such as a processor. The controller 120 may control overall operation of the elements of the specimen analysis apparatus 1 with control signals.

For example, in response to receiving a command from the user to output an analysis result, the controller 120 may send a control signal to control the output unit 11 to output the analysis result. In another example, the controller 120 may send a control signal to display the analysis result of the specimen on the display 21.

In still another example, the controller 120 may analyze the target material in the specimen based on the optical characteristics of the specimen detected by the optical detector 100. A method for determining a correction value for a concentration of the target material, which is performed by the controller 120, will now be described in detail.

The controller 120 may determine a concentration of the target material based on the absorbance. For example, as the concentration of the target material increases, the absorbance may increase or decrease depending on the reagent. The controller 120 may use the data stored in the memory 110 to derive a concentration of the target material from the absorbance in the container 45b.

First, the controller 120 may calculate concentrations of the target material in at least two sub-containers in order to determine a correction value for a concentration of the target material.

For example, a reagent with no internal standard material may be provided in the first container, and a reagent with the internal standard material of concentration $\alpha$ may be provided in the second container. Information regarding concentrations of the internal standard material provided in the respective sub-containers may be stored in the memory 110 or mapped to and stored in the identification information provided on the cartridge 40.

The controller 120 may determine optical signal values of the target material in the first and second containers. For example, the controller 120 may determine not only the absorbance, fluorescence, luminance but also concentrations in the respective first and second containers.

The controller 120 may then set the absorbance and concentration in a two dimensional (2D) coordinate system, as shown in FIG. 10A. In FIG. 10A, the x-axis represents the concentration of the target material, and the y-axis represents the absorbance of the target material. However, the y-axis is not limited to the absorbance of the target material but may represent the fluorescence or luminance of the target material, without being limited thereto.

The absorbance of the target material in the first and second containers correspond respectively to c and d. The controller 120 may then calculate concentration S of the target material in the first container based on the absorbance.

Information about the concentration $\alpha$ of the target material that increases due to the internal standard material provided in the second container in advance may be mapped to the identification information or stored in the memory 110. Accordingly, the controller 120 may calculate the concentration of the target material in the second container from the absorbance d, but also calculate the concentration $S+\alpha$ of the target material in the second container from the information stored in the identification information or the memory 110.

In the case of calculating the concentration of the target material in the second container from the absorbance d, the optical signal value may not be accurate as the term of validity (or effective life) expires. Accordingly, the controller 120 may use the concentration $\alpha$ determined from the memory 110 or the identification information to calculate the concentration of the target material in the second container, thereby more accurately determining a difference in concentration of the target material between the first and second containers.

Referring to FIG. 10A, in the graph, the coordinates of the optical signal value of the first container may be set to (S, c), and the coordinates of the optical signal value of the second container may be set to $(S+\alpha, d)$. In the following description, for the convenience of explanation, the coordinates of the optical signal values of the first and second containers are referred to as first and second coordinates, respectively.

Depending on the properties of the reagent, the absorbance c may be less than the absorbance *d* as shown in FIG. 10A, but alternatively, the absorbance c may be greater than the absorbance *d*. For example, depending on the properties of the reagent, the target material may come in brighter color or darker color according to a reaction with the reagent, without being limited thereto.

The controller 120 may determine an extent of change in the optical signal value using the coordinates set in the graph. For example, the controller 120 may generate a straight line that connects the first coordinates (S, c) and the

second coordinates (S+$\alpha$, d) as shown in FIG. 10B, and calculate a slope *a* of the straight line. The slope *a* refers to an inclination derived based on the extent of change in the optical signal value derived from the reaction between the specimen and the reagent in the container.

The information about the property of the reagent may include information relating to a concentration graph depending on the absorbance, as shown in FIGS. 11A and 11B. The specimen analysis apparatus 1 may compare the optical signal value derived in the container 45b with the optical signal value derived in an ideal state to compensate for or correct the optical signal value derived in the container 45b, thereby increasing the term of validity of the cartridge 40.

Referring to FIG. 11A, the change of concentration depending on the absorbance in may be constant with the slope *b* a certain range. In another example, referring to FIG. 11B, the slope *b1* representing the change of concentration depending on the absorbance may not be constant. The slopes *b* and *b1* are derived based on the stored data, e.g., the information about the property of the reagent, and correspond to slopes derived based on the extent of change in the optical signal value according to the difference between the internal standard materials in an ideal state. A method for determining a correction value when the slope is constant will now be described first.

In the case that the slope *b* is constant between concentrations S and S+$\alpha$, the controller 120 may determine a correction value using the slopes *a* and *b.* For example, the controller 120 may determine a correction ratio S' for the concentration of the target material as in the following equation 1:

$$S'=(b/a) \qquad\qquad (1)$$

Accordingly, the controller 120 may determine the corrected concentration S" of the target material as in the following equation 2:

$$S''=S \times S'=S(b/a) \qquad\qquad (2)$$

The controller 120 may correct the optical signal value of the target material, thereby increasing the term of validity of the cartridge 40.

As shown in the graph of FIG. 11B, the slope *b1* between the concentrations S and S+$\alpha$ may not be constant. In this case, the controller 120 may determine a correction value using various methods.

For example, the controller 120 may calculate an average slope *b11* between the concentrations S and S+$\alpha$, and use the average slope *b11* to determine a corrected concentration of the target material as in the following equation 3:

$$S''=S \times (b11/a) \qquad\qquad (3)$$

In still another example, the controller 120 may use a tangent of the plot at the concentration S, i.e., a slope *b12* of the plot at the concentration S, to determine a corrected concentration of the target material as in the following equation 4:

$$S''=S \times (b12/a) \qquad\qquad (4)$$

As described above, if the container includes a plurality of sub-containers, e.g., three sub-containers, a specimen is injected to the first, second, and third containers and may react with the reagents. In this case, the concentration of the internal standards material provided in advance in the third container may be higher than those in the first and second containers. For example, the concentration of the target material that increases due to the internal standard material injected to the third container may be $\beta$ ($\beta > \alpha$).

A slope between the first and second containers, i.e., a slope between the specimen concentrations S and S+$\alpha$, may or may not be the same as the slope between the second and third containers, i.e., the slope between the specimen concentrations S+$\alpha$ and S+$\beta$. For example, referring to FIG. 12, the slopes *a2* and *a3* may or may not be the same.

The controller 120 may calculate the slope *b2* of a region between the specimen concentrations S and S+$\alpha$ and the slope *b3* of a region between the specimen concentrations S+$\alpha$ and S+$\beta$ based on the information about the property of the reagent, as shown in FIG. 13.

The slope *b2* of the region between the specimen concentrations S and S+$\alpha$ may be an average slope of the region or a slope at the specimen concentration S. The slope *b3* of the region between the specimen concentrations S+$\alpha$ and S+$\beta$ may also be an average slope of the region, or a slope at the specimen concentration S+$\alpha$, without being

limited thereto.

The controller may determine a correction value for each region, and use the correction value to calculate a corrected concentration of the target material for the region. Accordingly, the controller 120 may provide the trend of changes in the optical signal value, which are expected more accurately, i.e., a graph representing changes of the optical signal value, thereby making more accurate diagnosis on the target subject.

The controller 120 may use the optical signal values in all the sub-containers to correct the trend of changes in the target material, or use only the optical signal values in a particular range to correct the trend of changes in the target material.

The controller 120 may use only the optical signal values in a predetermined measurement range, e.g., in a dynamic range of the optical detector 100 to correct the trend of changes of the target material.

An optical signal value outside of the measurement range may have a significant error. Accordingly, the controller 120 may determine whether the optical signal values derived from the plurality of the sub-containers are in a reliable range, and based on the determination result, may select an optical signal value to be used in correcting a concentration. In other words, the controller 120 may not use all the optical signal values derived from all the sub-containers but select one of the sub-containers, in which an optical signal value is derived from the set measurement range, and use the optical signal value in the selected sub-container, thereby leaving out optical signal values with significant errors.

A normal measurement range may be set in advance using at least one of the optical signal values. For example, a first measurement range DR1 set in advance may be set to a particular concentration range, as shown in FIG. 14A. In this regard, the controller 120 may not use the optical signal value for the third container for correction, which is out of the first measurement range DR1.

In another example, a second measurement range DR2 set in advance may be set to a particular absorbance range, as shown in FIG. 14B. In this regard, the controller 120 may not use the optical signal value for the third container for correction, which is out of the second measurement range DR.

The controller 120 may display a user interface on the display 21, which is implemented to include at least one of icons that may receive various control instructions, the corrected concentration of the target material, and plots of changes in the corrected concentration of the target material. The user interface refers to an environment in which the user may be able to control the elements and program of the specimen analysis apparatus 1 and obtain the various information more easily. The user interface as herein used may be a GUI that graphically implements screens to be displayed on the display 21 in order to more conveniently perform exchange of various information between the user and the specimen analysis apparatus 1.

For example, the GUI may be implemented to display icons or buttons in some area on the screen displayed on the display 21 to receive various control instructions from the user more easily, and display at least one widget or pop-up message in some other area to display various information.

FIG. 15 shows a user interface screen displayed on a display, which is configured to provide a corrected concentration of a target material, and FIG. 16 shows a user interface screen displayed on a display, which is configured to provide a corrected concentration of a target material and a plot of the corrected concentration of the target material. configured to include the name of a subject to be analyzed, 'Jordan', and the time stamp of the test, ' 12/June/2012 09:50 AM', as shown in FIG. 15. Furthermore, the user interface may be configured to include a pop-up message 1420 saying contents of a cartridge number, Q01 and a corrected concentration of the target material, K:8.5. There are no limitations on how to configure the user interface. For example, the user interface may include an icon 1410 to go back to the main screen.

Moreover, the controller 120 may display a user interface on the display 21, which is configured to provide not only a corrected concentration of the target material but also a pop-up message 1430 having a plot of changes of the corrected concentration of the target material, as shown in FIG. 16. Operation flows of the specimen analysis apparatus 1 will now be briefly described.

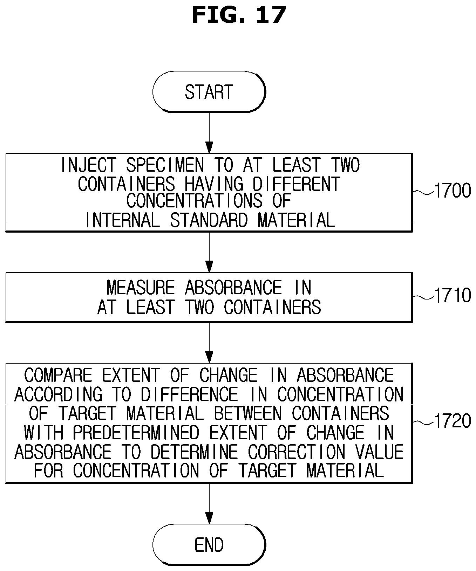

FIG. 17 is a flowchart illustrating operation of a specimen analysis apparatus for determining a correction value for a concentration of a target material.

A cartridge may be inserted into a specimen analysis apparatus. For example, the specimen analysis apparatus 1 may include the door frame 23 (see FIG. 1) equipped with the install member 32 (see FIG. 2) on which the cartridge 40 (also see FIG. 2) for containing various reagents may be mounted, allowing the cartridge 40 to be mounted on the install member 32 by insertion.

At least two containers formed in the cartridge 40 may contain reagents in advance, which include an internal standard material with predetermined concentrations.

In the specimen analysis apparatus 1, a specimen may be injected into the at least two containers having the internal standard material with different concentrations through the specimen supplier 42 (see FIG. 2) of the cartridge 40, in operation 1700.

The specimen injected through the specimen supplier 42 may flow into the container 45b along the fluid path 47c (see FIG. 7) and make a reaction with the reagent. The specimen injection apparatus 1 may detect optical characteristics from the reaction between the specimen and the reagent in the container with the optical detector.

The specimen analysis apparatus may use the optical detector to measure the absorbance of the target material in the at least two containers, in operation 1710. Since the at least two containers contain the internal standard material with different concentrations, concentrations of the target material in the at least two containers may be different and the measured absorbance may also be different.

The specimen analysis apparatus may then determine an extent of change in the absorbance according to the difference of concentrations of the target material between the containers. The difference of concentrations of the target material between the containers may also be derived from the absorbance, or from the identification information provided on the cartridge 40 or the information about the property of the reagent stored in the memory.

The specimen analysis apparatus 1 may determine a correction value for a concentration of the target material by comparing the extent of change in absorbance according to the difference of concentrations of the target material between the containers with the extent of change in absorbance based on the difference of concentrations derived from the identification information attached to the cartridge 40 or the information about the property of the reagent stored in the memory, in operation 1720.

The extent of change in absorbance according to the difference of concentrations of the target material between containers is called a measured calibration curve, and the extent of change in absorbance based on the difference of concentrations derived from the identification information provided on the cartridge 40 or the information about the property of the reagent stored in the memory is called a factory calibration curve.

At the time the cartridge is produced, reagents may be injected to a plurality of containers. In this regard, it is more likely that the reagent fails to normally react with the specimen as the description may change with the passing of time.

Further, it is also more likely that the reagent fails to make a normal reaction with the specimen, if the provided reagent does not reach a fixed quantity, or if an interfering material is provided in the container in the process of manufacturing the cartridge.

The specimen analysis apparatus may enable more accurate diagnosis of the subject as well as increase a period of usage of the cartridge, by comparing the measured calibration curve with the factory calibration curve to correct the optical signal value of a target material. Operation flows of the specimen analysis apparatus for correcting the optical signal value of a target material using a reliable optical signal value will now be descried briefly.

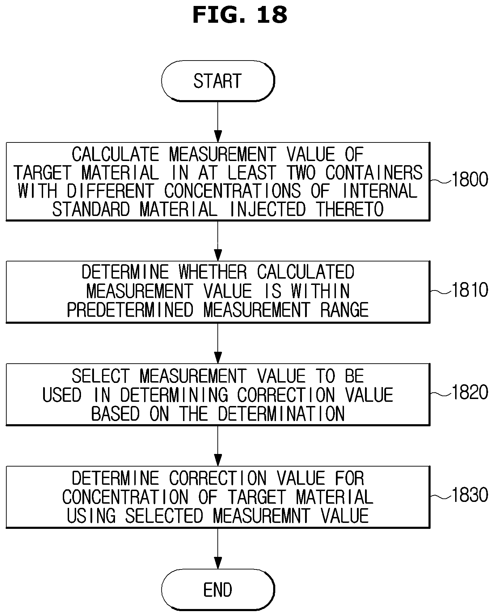

FIG. 18 is a flowchart illustrating operation of a specimen analysis apparatus for determining a correction value by selecting an optical signal value from within a normal measurement range.

The specimen analysis apparatus calculates optical signal values of the target material in at least two containers having an internal standard material injected with different concentrations, in operation 1800. The optical signal value of the target material may include at least one of concentration, luminance, fluorescence, and absorbance of the target material. Details of how to calculate the optical signal value of the target material were described above, so the description will now be omitted.

The specimen analysis apparatus determines whether the optical signal value of the target material is within a set measurement range, in operation 1810. The set measurement range refers to a range used to determine whether the optical signal value of the target material is in a reliable range. The set measurement range may be stored in the memory of the specimen analysis apparatus, or may be mapped to and stored in the identification information. The set measurement range may be set based on concentration values or absorbance, as described above, without being limited thereto. The specimen analysis apparatus may enable more accurate diagnosis of the subject by correcting a concentration of the target material using a reliable optical signal value.

The specimen analysis apparatus determines whether the optical signal value of the target material is within the set measurement range, and based on the determination, selects an optical signal value to be used in determining a correction value, in operation 1820. For example, referring to FIG. 14B, the specimen analysis apparatus may leave out optical signal values for the third container, which are out of the set second measurement range DR2 in determining the correction value.

The specimen analysis apparatus determines a correction value for a concentration of the target material using the selected at least one optical signal value, in operation 1830. The specimen analysis apparatus may generate a measured calibration curve using the optical signal values in the container, and determine a correction value by comparing the measured calibration curve and the factory calibration curve. This was described above, so the detailed description thereof will be omitted.

Accordingly, the specimen analysis apparatus may increase the terms of validity of the cartridge, and increase reliability of the product itself by enabling more accurate diagnosis of the subject.

**Claims**

1. A specimen analysis apparatus (1) for determining a concentration of a target material in a specimen, comprising:

a cartridge (40), comprising at least two containers (45b), each container (45b) containing a reagent configured to react with the target material in the specimen to measure the concentration of the target material, wherein each reagent further includes a predetermined concentration of internal standard material containing the target material, wherein the internal standard material is configured to release the target material while being dissolved during a process of mixing with the specimen and wherein the concentrations of the internal standard materials in the reagents of the at least two containers (45b) are different;

an optical detector unit (100) for detecting optical characteristics from a reaction between the target material in the specimen and the released target material in the internal standard materials with the reagents in the at least two containers (45b) and providing optical signal values;

a controller (120) configured to:

derive a measured calibration curve from an extent of a change in the optical signal value of the target material measured in the at least two containers (45b);

**characterized in that** the controller (120) is further configured to:

obtain, from identification information provided on the cartridge and/or from information about the property of the reagent stored in a memory of the apparatus, a factory calibration curve indicating a predetermined extent of a change in the optical signal value of the target material in the at least two containers;

determine, based on a comparison of the measured calibration curve with the factory calibration curve, a correction value for calculation of the concentration of the target material; and

determine a corrected concentration of the target material based on the correction value.

2. The specimen analysis apparatus (1) of claim 1,
wherein one of the at least two containers (45b) has no internal standard material injected thereto, and the other of the at least two containers (45b) has an internal standard material injected thereto with a predetermined concentration.

3. The specimen analysis apparatus (1) of claim 1,
wherein the extent of the change in the optical signal value of the target material comprises an extent of a change in at least one of an absorbance, a fluorescence, and a luminance according to a difference of concentrations of the target material in the at least two containers (45b).

4. The specimen analysis apparatus (1) of claim 1,
wherein the controller (120) is configured to calculate a slope of a change in an absorbance based on an absorbance of the target material measured from one of the at least two containers (45b), an absorbance of the target material measured from the other one, and a difference in concentrations of the target material between the at least two containers (45b), the correction value for the concentration of the target material being determined using the calculated slope of the change in the absorbance.

5. The specimen analysis apparatus (1) of claim 1,

wherein the controller (120) is configured to calculate an amount of a change in an absorbance based on an absorbance of the target material measured from one of the at least two containers (45b), an absorbance of the target material measured from the other one, and a difference in concentrations of the target material between the at least two containers (45b), and

wherein the measured calibration curve and the factory calibration curve are derived from the calculated amount of change in absorbance, the correction value for the corrected concentration of the target material being determined using the calculated amount of change in absorbance based on the difference in concentrations of the target material between the at least two containers.

6. The specimen analysis apparatus (1) of claim 1,
wherein the controller (120) is configured to calculate a slope of a change in a luminance based on a luminance of the target material measured from one of the at least two containers (45b), a luminance of the target material measured from the other one, and a difference in concentrations of the target material between the at least two containers (45b), the correction value for the corrected concentration of the target material being determined using the calculated slope of change in luminance.

7. The specimen analysis apparatus (1) of claim 1,
wherein the controller (120) is configured to calculate a slope of change in a fluorescence based on a fluorescence of the target material measured from one of the at least two containers (45b), a fluorescence of the target material measured from the other one, and a difference in concentrations of the target material between the at least two containers (45b), the correction value for the corrected concentration of the target material being determined using the calculated slope of change in fluorescence.

8. The specimen analysis apparatus (1) of claim 1,
wherein the controller (120) is configured to determine at least one region between at least two specimen concentrations in the measured calibration curve corresponding to a difference in concentrations of the target material between the at least two containers (45b) and an extent of a change in optical signal value of the target material for each region, and compare the extent of the change in optical signal value of the target material for each region with the factory calibration curve derived from the predetermined extent of a change in optical signal value of the target material for the region to determine a correction value for a concentration of the target material for each region.

9. The specimen analysis apparatus (1) of claim 1, comprising at least one of the following features:

the controller (120) is configured to create a plot of concentration changes from an absorbance of the target material based on the correction value for a concentration of the target material,
a predetermined extent of change in optical signal value of the target material is stored in a memory (110) or mapped to and stored in identification information attached to the cartridge (40), and
the controller (120) is configured to determine whether optical signal values of the target material measured from the at least two containers (45b) are within a predetermined measurement range, and based on the determination, determine whether to use the optical signal values in determining the correction value for the concentration of the target material.

10. A measurement method for determining a concentration of a target material in a specimen, the method comprising:

receiving the specimen in at least two containers (45b), each container (45b) containing a reagent configured to react with the target material in the specimen to measure the concentration of the target material, wherein each reagent further includes a predetermined concentration of internal standard material containing the target material, wherein the internal standard material is configured to release the target material while being dissolved during a process of mixing with the specimen and wherein the concentrations of the internal standard materials in the reagents of the at least two containers (45b) are different;
detecting optical characteristics from a reaction between the target material in the specimen and the internal standard materials with the reagents in the at least two containers (45b) and providing optical signal values;
deriving a measured calibration curve from an extent of a change in the optical signal value of the target material measured in the at least two containers (45b);
**characterized in that** the method further comprises:

obtaining, from identification information provided on the cartridge and/or from information about the property of the reagent stored in a memory of the apparatus, a factory calibration curve indicating a predetermined extent of a change in the optical signal value of the target material in the at least two containers;
determining, based on a comparison of the measured calibration curve with the factory calibration curve, a correction value for calculation of the concentration of the target material; and
determining a corrected concentration of the target material based on the correction value.

11. The method of claim 10,
wherein the determining the correction value for the concentration of the target material comprises:
calculating a slope of change in an absorbance based on an absorbance of the target material measured from one of the at least two containers (45b), an absorbance of the target material measured from the other one, and a difference in concentrations of the target material between the at least two containers (45b), the correction value for the concentration of the target material being determined using the calculated slope of the change in absorbance.

12. The method of claim 10,
wherein determining the correction value for the concentration of the target material comprises:
calculating an amount of change in an absorbance based on an absorbance of the target material measured from one of the at least two containers (45b), an absorbance of the target material measured from the other one, and a difference in concentrations of the target material between the at least two containers (45b), the correction value for the concentration of the target material being determined using the calculated amount of change in the absorbance according to the difference in concentration of the target material and by comparing the calculated change in absorbance with a predetermined change in absorbance.

13. The method of claim 10,
wherein the determining the correction value for the concentration of the target material comprises:
calculating a slope of a change in a luminance based on a luminance of the target material measured from one of the at least two containers (45b), a luminance of the target material measured from the other one, and a difference in concentrations of the target material between the at least two containers (45b), the correction value for the concentration of the target material being determined using the calculated slope of change in luminance.

14. The method of claim 10,
wherein the determining the correction value for the concentration of the target material comprises:
calculating a slope of change in a fluorescence based on a fluorescence of the target material measured from one of the at least two containers (45b), a fluorescence of the target material measured from the other one, and a difference in concentrations of the target material between the at least two containers (45b), the correction value for the concentration of the target material being determined using the calculated slope of change in fluorescence.

15. The method of claim 10, further comprising steps of:

determining at least one region between at least two specimen concentrations in the measured calibration curve corresponding to a difference in concentrations of the target material between the at least two containers (45b) and an extent of a change in an optical signal value of the target material for each region, and
comparing the extent of change in the optical signal value of the target material for each region with the factory calibration curve derived from the predetermined extent of a change in the optical signal value of the target material for the region, to determine a correction value for a concentration of the target material for each region; and/or
creating a plot of concentration changes according to an absorbance of the target material based on the determined correction value for the concentration of the target material; and/or
determining whether optical signal values of the target material measured from the at least two containers (45b) are within a predetermined measurement range, and based on the determination, determining whether to use the optical signal values in determining the correction value for the concentration of the target material.

**Patentansprüche**

1. Probenanalyseeinrichtung (1) zum Bestimmen einer Konzentration eines Zielmaterials in einer Probe, umfassend:

eine Kartusche (40), die mindestens zwei Behälter (45b) umfasst, wobei jeder Behälter (45b) ein Reagenz enthält, das so konfiguriert ist, dass es mit dem Zielmaterial in der Probe reagiert, um die Konzentration des Zielmaterials zu messen, wobei jedes Reagenz weiter eine vorbestimmte Konzentration an internem Standardmaterial beinhaltet, das das Zielmaterial enthält, wobei das interne Standardmaterial so konfiguriert ist, dass es das Zielmaterial freisetzt, während es während eines Prozesses des Mischens mit der Probe aufgelöst wird, und wobei sich die Konzentrationen der internen Standardmaterialien in den Reagenzien der mindestens zwei Behälter (45b) unterscheiden;
eine optische Detektoreinheit (100) zum Detektieren von optischen Merkmalen anhand einer Reaktion zwischen dem Zielmaterial in der Probe und dem freigesetzten Zielmaterial in den internen Standardmaterialien mit den Reagenzien in den mindestens zwei Behältern (45b), und Bereitstellen von optischen Signalwerten;
eine Steuerung (120), die so konfiguriert ist, dass sie:

eine gemessene Kalibrierungskurve anhand eines Ausmaßes einer Änderung des optischen Signalwerts des Zielmaterials, der in den mindestens zwei Behältern (45b) gemessen wird, ableitet;
**dadurch gekennzeichnet, dass** die Steuerung (120) weiter so konfiguriert ist, dass sie:

anhand von Identifikationsinformationen, die auf der Kartusche bereitgestellt sind, und/oder anhand von Informationen über die Eigenschaft des Reagens, die in einem Speicher der Einrichtung gespeichert sind, eine Werkskalibrierungskurve erhält, die ein vorbestimmtes Ausmaß einer Änderung des optischen Signalwerts des Zielmaterials in den mindestens zwei Behältern angibt;
auf Basis eines Vergleichs der gemessenen Kalibrierungskurve mit der Werkskalibrierungskurve einen Korrekturwert zum Berechnen der Konzentration des Zielmaterials bestimmt; und
eine korrigierte Konzentration des Zielmaterials auf Basis des Korrekturwerts bestimmt.

**2.** Probenanalyseeinrichtung (1) nach Anspruch 1,
wobei einer der mindestens zwei Behälter (45b) kein internes Standardmaterial aufweist, das ihm injiziert wird, und der andere der mindestens zwei Behälter (45b) ein internes Standardmaterial aufweist, das ihm mit einer vorbestimmten Konzentration injiziert wird.

**3.** Probenanalyseeinrichtung (1) nach Anspruch 1,
wobei das Ausmaß der Änderung des optischen Signalwerts des Zielmaterials ein Ausmaß einer Änderung mindestens eines von einem Absorptionsgrad, einer Fluoreszenz und einer Leuchtdichte gemäß einer Differenz der Konzentrationen des Zielmaterials in den mindestens zwei Behältern (45b) umfasst.

**4.** Probenanalyseeinrichtung (1) nach Anspruch 1,
wobei die Steuerung (120) so konfiguriert ist, dass sie eine Steigung einer Änderung eines Absorptionsgrades auf Basis eines Absorptionsgrades des Zielmaterials, der anhand eines der mindestens zwei Behälter (45b) gemessen wird, eines Absorptionsgrades des Zielmaterials, der anhand des anderen gemessen wird, und einer Differenz der Konzentrationen des Zielmaterials zwischen den mindestens zwei Behältern (45b) berechnet, wobei der Korrekturwert für die Konzentration des Zielmaterials unter Verwendung der berechneten Steigung der Änderung des Absorptionsgrades bestimmt wird.

**5.** Probenanalyseeinrichtung (1) nach Anspruch 1,

wobei die Steuerung (120) so konfiguriert ist, dass sie auf Basis eines Absorptionsgrades des Zielmaterials, der anhand eines der mindestens zwei Behälter (45b) gemessen wird, eines Absorptionsgrades des Zielmaterials, der anhand des anderen gemessen wird, und einer Differenz der Konzentrationen des Zielmaterials zwischen den mindestens zwei Behältern (45b) einen Betrag einer Änderung eines Absorptionsgrades berechnet, und
wobei die gemessene Kalibrierungskurve und die Werkskalibrierungskurve anhand des berechneten Änderungsbetrags des Absorptionsgrades abgeleitet werden, wobei der Korrekturwert für die korrigierte Konzentration des Zielmaterials unter Verwendung des berechneten Änderungsbetrags des Absorptionsgrades auf Basis der Differenz der Konzentrationen des Zielmaterials zwischen den mindestens zwei Behältern bestimmt wird.

**6.** Probenanalyseeinrichtung (1) nach Anspruch 1,
wobei die Steuerung (120) so konfiguriert ist, dass sie eine Steigung einer Änderung einer Leuchtdichte auf Basis einer Leuchtdichte des Zielmaterials, die anhand eines der mindestens zwei Behälter (45b) gemessen wird, einer Leuchtdichte des Zielmaterials, die anhand des anderen gemessen wird, und einer Differenz der Konzentrationen des Zielmaterials zwischen den mindestens zwei Behältern (45b) berechnet, wobei der Korrekturwert für die korrigierte Konzentration des Zielmaterials unter Verwendung der berechneten Steigung der Änderung der Leuchtdichte bestimmt wird.

**7.** Probenanalyseeinrichtung (1) nach Anspruch 1,
wobei die Steuerung (120) so konfiguriert ist, dass sie eine Steigung der Änderung einer Fluoreszenz auf Basis einer Fluoreszenz des Zielmaterials, die anhand eines der mindestens zwei Behälter (45b) gemessen wird, einer Fluoreszenz des Zielmaterials, die anhand des anderen gemessen wird, und einer Differenz der Konzentrationen des Zielmaterials zwischen den mindestens zwei Behältern (45b) berechnet, wobei der Korrekturwert für die korrigierte Konzentration des Zielmaterials unter Verwendung der berechneten Steigung der Änderung der Fluoreszenz bestimmt wird.

**8.** Probenanalyseeinrichtung (1) nach Anspruch 1,
wobei die Steuerung (120) so konfiguriert ist, dass sie mindestens einen Bereich zwischen mindestens zwei Probenkonzentrationen in der gemessenen Kalibrierungskurve, der einer Differenz der Konzentrationen des Zielmaterials zwischen den mindestens zwei Behältern (45b) entspricht, und ein Ausmaß einer Änderung des optischen Signalwerts des Zielmaterials für jeden Bereich bestimmt, und das Ausmaß der Änderung des optischen Signalwerts des Zielmaterials für jeden Bereich mit der Werkskalibrierungskurve vergleicht, die anhand des vorbestimmten Ausmaßes einer Änderung des optischen Signalwerts des Zielmaterials für den Bereich abgeleitet wird, um einen Korrekturwert für eine Konzentration des Zielmaterials für jeden Bereich zu bestimmen.

9.  Probenanalyseeinrichtung (1) nach Anspruch 1, die mindestens eines der folgenden Merkmale umfasst:

    die Steuerung (120) ist so konfiguriert, dass sie anhand eines Absorptionsgrades des Zielmaterials auf Basis des Korrekturwerts für eine Konzentration des Zielmaterials eine Darstellung von Konzentrationsänderungen erzeugt,
    ein vorbestimmtes Ausmaß der Änderung des optischen Signalwerts des Zielmaterials ist in einem Speicher (110) gespeichert oder Identifikationsinformationen, die an der Kartusche (40) angebracht sind, zugeordnet und in diesen gespeichert, und
    die Steuerung (120) ist so konfiguriert, dass sie bestimmt, ob optische Signalwerte des Zielmaterials, die anhand der mindestens zwei Behälter (45b) gemessen werden, innerhalb eines vorbestimmten Messbereichs liegen, und auf Basis der Bestimmung bestimmt, ob die optischen Signalwerte beim Bestimmen des Korrekturwerts für die Konzentration des Zielmaterials verwendet werden sollen.

10. Messverfahren zum Bestimmen einer Konzentration eines Zielmaterials in einer Probe, wobei das Verfahren umfasst:

    Empfangen der Probe in mindestens zwei Behältern (45b), wobei jeder Behälter (45b) ein Reagenz enthält, das so konfiguriert ist, dass es mit dem Zielmaterial in der Probe reagiert, um die Konzentration des Zielmaterials zu messen, wobei jedes Reagenz weiter eine vorbestimmte Konzentration an internem Standardmaterial beinhaltet, das das Zielmaterial enthält, wobei das interne Standardmaterial so konfiguriert ist, dass es das Zielmaterial freisetzt, während es während eines Prozesses des Mischens mit der Probe aufgelöst wird, und wobei sich die Konzentrationen der internen Standardmaterialien in den Reagenzien der mindestens zwei Behälter (45b) unterscheiden;
    Detektieren von optischen Merkmalen anhand einer Reaktion zwischen dem Zielmaterial in der Probe und den internen Standardmaterialien mit den Reagenzien in den mindestens zwei Behältern (45b), und Bereitstellen von optischen Signalwerten;
    Ableiten einer gemessenen Kalibrierungskurve anhand eines Ausmaßes einer Änderung des optischen Signalwerts des Zielmaterials, der in den mindestens zwei Behältern (45b) gemessen wird;
    **dadurch gekennzeichnet, dass** das Verfahren weiter umfasst:

    Erhalten, anhand von Identifikationsinformationen, die auf der Kartusche bereitgestellt sind, und/oder anhand von Informationen über die Eigenschaft des Reagens, die in einem Speicher der Einrichtung gespeichert sind, einer Werkskalibrierungskurve, die ein vorbestimmtes Ausmaß einer Änderung des optischen Signalwerts des Zielmaterials in den mindestens zwei Behältern angibt;
    Bestimmen, auf Basis eines Vergleichs der gemessenen Kalibrierungskurve mit der Werkskalibrierungskurve, eines Korrekturwerts zum Berechnen der Konzentration des Zielmaterials; und
    Bestimmen einer korrigierten Konzentration des Zielmaterials auf Basis des Korrekturwerts.

11. Verfahren nach Anspruch 10,
    wobei das Bestimmen des Korrekturwerts für die Konzentration des Zielmaterials umfasst:
    Berechnen einer Steigung der Änderung eines Absorptionsgrades auf Basis eines Absorptionsgrades des Zielmaterials, der anhand eines der mindestens zwei Behälter (45b) gemessen wird, eines Absorptionsgrades des Zielmaterials, der anhand des anderen gemessen wird, und einer Differenz der Konzentrationen des Zielmaterials zwischen den mindestens zwei Behältern (45b), wobei der Korrekturwert für die Konzentration des Zielmaterials unter Verwendung der berechneten Steigung der Änderung des Absorptionsgrades bestimmt wird.

12. Verfahren nach Anspruch 10,
    wobei das Bestimmen des Korrekturwerts für die Konzentration des Zielmaterials umfasst:
    Berechnen eines Betrags der Änderung eines Absorptionsgrades auf Basis eines Absorptionsgrades des Zielmaterials, der anhand eines der mindestens zwei Behälter (45b) gemessen wird, eines Absorptionsgrades des Zielmaterials, der anhand des anderen gemessen wird, und einer Differenz der Konzentrationen des Zielmaterials zwischen den mindestens zwei Behältern (45b), wobei der Korrekturwert für die Konzentration des Zielmaterials unter Verwendung des berechneten Änderungsbetrags des Absorptionsgrades gemäß der Differenz der Konzentrationen des Zielmaterials, und durch Vergleichen der berechneten Änderung des Absorptionsgrades mit einer vorbestimmten Änderung des Absorptionsgrades bestimmt wird.

13. Verfahren nach Anspruch 10,
    wobei das Bestimmen des Korrekturwerts für die Konzentration des Zielmaterials umfasst:
    Berechnen einer Steigung einer Änderung einer Leuchtdichte auf Basis einer Leuchtdichte des Zielmaterials, die anhand eines der mindestens zwei Behälter (45b) gemessen wird, einer Leuchtdichte des Zielmaterials, die anhand des anderen gemessen wird, und einer Differenz der Konzentrationen des Zielmaterials zwischen den mindestens zwei Behältern (45b), wobei der Korrekturwert für die Konzentration des Zielmaterials unter Verwendung der berechneten Steigung der Änderung der Leuchtdichte bestimmt wird.

**14.** Verfahren nach Anspruch 10,
wobei das Bestimmen des Korrekturwerts für die Konzentration des Zielmaterials umfasst:
Berechnen einer Steigung der Änderung einer Fluoreszenz auf Basis einer Fluoreszenz des Zielmaterials, die anhand eines der mindestens zwei Behälter (45b) gemessen wird, einer Fluoreszenz des Zielmaterials, die anhand des anderen gemessen wird, und einer Differenz der Konzentrationen des Zielmaterials zwischen den mindestens zwei Behältern (45b), wobei der Korrekturwert für die Konzentration des Zielmaterials unter Verwendung der berechneten Steigung der Änderung der Fluoreszenz bestimmt wird.

**15.** Verfahren nach Anspruch 10, das weiter die folgenden Schritte umfasst:

Bestimmen mindestens eines Bereichs zwischen mindestens zwei Probenkonzentrationen in der gemessenen Kalibrierungskurve, der einer Differenz der Konzentrationen des Zielmaterials zwischen den mindestens zwei Behältern (45b) entspricht, und eines Ausmaßes einer Änderung eines optischen Signalwerts des Zielmaterials für jeden Bereich, und

Vergleichen des Ausmaßes der Änderung des optischen Signalwerts des Zielmaterials für jeden Bereich mit der Werkskalibrierungskurve, die anhand des vorbestimmten Ausmaßes einer Änderung des optischen Signalwerts des Zielmaterials für den Bereich abgeleitet wird, um einen Korrekturwert für eine Konzentration des Zielmaterials für jeden Bereich zu bestimmen; und/oder

Erzeugen einer Darstellung von Konzentrationsänderungen gemäß einem Absorptionsgrad des Zielmaterials auf Basis des bestimmten Korrekturwerts für die Konzentration des Zielmaterials; und/oder

Bestimmen, ob optische Signalwerte des Zielmaterials, die anhand der mindestens zwei Behälter (45b) gemessen werden, innerhalb eines vorbestimmten Messbereichs liegen, und Bestimmen, auf Basis der Bestimmung, ob die optischen Signalwerte beim Bestimmen des Korrekturwerts für die Konzentration des Zielmaterials verwendet werden sollen.

## Revendications

**1.** Appareil d'analyse de spécimen (1) pour déterminer une concentration d'un matériau cible dans un spécimen, comprenant :

une cartouche (40) comprenant au moins deux récipients (45b), chaque récipient (45b) contenant un réactif configuré pour réagir avec le matériau cible dans le spécimen afin de mesurer la concentration du matériau cible, dans lequel chaque réactif comprend en outre une concentration prédéterminée de matériau étalon interne contenant le matériau cible, dans lequel le matériau étalon interne est configuré pour libérer le matériau cible tout en étant dissous pendant un processus de mélange avec le spécimen et dans lequel les concentrations des matériaux étalons internes dans les réactifs des au moins deux récipients (45b) sont différentes ;
une unité de détection optique (100) pour détecter des caractéristiques optiques à partir d'une réaction entre le matériau cible dans le spécimen et le matériau cible libéré dans les matériaux étalons internes avec les réactifs dans les au moins deux récipients (45b) et fournir des valeurs de signaux optiques ;
un contrôleur (120) configuré pour :

dériver une courbe de calibration mesurée à partir d'une étendue d'un changement dans la valeur de signal optique du matériau cible mesurée dans les au moins deux récipients (45b) ;
**caractérisé en ce que** le contrôleur (120) est en outre configuré pour :

obtenir, à partir d'informations d'identification fournies sur la cartouche et/ou à partir d'informations sur la propriété du réactif stockées dans une mémoire de l'appareil, une courbe de calibration d'usine indiquant une étendue prédéterminée d'un changement dans la valeur de signal optique du matériau cible dans les au moins deux récipients ;
déterminer, sur la base d'une comparaison de la courbe de calibration mesurée avec la courbe de calibration d'usine, une valeur de correction pour le calcul de la concentration du matériau cible ; et
déterminer une concentration corrigée du matériau cible sur la base de la valeur de correction.

2.  Appareil d'analyse de spécimen (1) de la revendication 1,
    dans lequel l'un des au moins deux récipients (45b) n'a pas de matériau étalon interne injecté dans celui-ci, et l'autre des au moins deux récipients (45b) a un matériau étalon interne injecté dans celui-ci avec une concentration prédéterminée.

3.  Appareil d'analyse de spécimen (1) de la revendication 1,
    dans lequel l'étendue du changement dans la valeur de signal optique du matériau cible comprend une étendue d'un changement d'au moins l'une parmi une absorbance, une fluorescence et une luminance en fonction d'une différence de concentrations du matériau cible dans les au moins deux récipients (45b).

4.  Appareil d'analyse de spécimen (1) de la revendication 1,
    dans lequel le contrôleur (120) est configuré pour calculer une pente d'un changement dans une absorbance sur la base d'une absorbance du matériau cible mesurée à partir de l'un des au moins deux récipients (45b), d'une absorbance du matériau cible mesurée à partir de l'autre et d'une différence de concentrations du matériau cible entre les au moins deux récipients (45b), la valeur de correction pour la concentration du matériau cible étant déterminée en utilisant la pente calculée du changement dans l'absorbance.

5.  Appareil d'analyse de spécimen (1) de la revendication 1,

    dans lequel le contrôleur (120) est configuré pour calculer une quantité de changement dans une absorbance sur la base d'une absorbance du matériau cible mesurée à partir de l'un des au moins deux récipients (45b), d'une absorbance du matériau cible mesurée à partir de l'autre, et d'une différence de concentrations du matériau cible entre les au moins deux récipients (45b), et
    dans lequel la courbe de calibration mesurée et la courbe de calibration d'usine sont dérivées de la quantité calculée de changement dans l'absorbance, la valeur de correction pour la concentration corrigée du matériau cible étant déterminée en utilisant la quantité calculée de changement dans l'absorbance sur la base de la différence de concentrations du matériau cible entre les au moins deux récipients.

6.  Appareil d'analyse de spécimen (1) de la revendication 1,
    dans lequel le contrôleur (120) est configuré pour calculer une pente d'un changement dans une luminance sur la base d'une luminance du matériau cible mesurée à partir de l'un des au moins deux récipients (45b), d'une luminance du matériau cible mesurée à partir de l'autre et d'une différence de concentrations du matériau cible entre les au moins deux récipients (45b), la valeur de correction pour la concentration corrigée du matériau cible étant déterminée en utilisant la pente calculée de changement dans la luminance.

7.  Appareil d'analyse de spécimen (1) de la revendication 1,
    dans lequel le contrôleur (120) est configuré pour calculer une pente de changement dans une fluorescence sur la base d'une fluorescence du matériau cible mesurée à partir de l'un des au moins deux récipients (45b), d'une fluorescence du matériau cible mesurée à partir de l'autre, et d'une différence de concentrations du matériau cible entre les au moins deux récipients (45b), la valeur de correction pour la concentration corrigée du matériau cible étant déterminée en utilisant la pente calculée de changement dans la fluorescence.

8.  Appareil d'analyse de spécimen (1) de la revendication 1,
    dans lequel le contrôleur (120) est configuré pour déterminer au moins une région entre au moins deux concentrations de spécimen dans la courbe de calibration mesurée correspondant à une différence de concentrations du matériau cible entre les au moins deux récipients (45b) et une étendue d'un changement dans la valeur de signal optique du matériau cible pour chaque région, et comparer l'étendue du changement dans la valeur de signal optique du matériau cible pour chaque région avec la courbe de calibration d'usine dérivée de l'étendue prédéterminée d'un changement dans la valeur de signal optique du matériau cible pour la région afin de déterminer une valeur de correction pour une concentration du matériau cible pour chaque région.

9.  Appareil d'analyse de spécimen (1) de la revendication 1, comprenant au moins l'une des caractéristiques suivantes :

    le contrôleur (120) est configuré pour créer un tracé des changements de concentration à partir d'une absorbance du matériau cible sur la base de la valeur de correction pour une concentration du matériau cible,
    une étendue prédéterminée de changement dans la valeur de signal optique du matériau cible est stockée dans une mémoire (110) ou mappée à et stockée dans des informations d'identification attachées à la cartouche (40), et

le contrôleur (120) est configuré pour déterminer si des valeurs de signaux optiques du matériau cible mesurées à partir des au moins deux récipients (45b) se trouvent dans une plage de mesure prédéterminée, et sur la base de la détermination, déterminer s'il faut utiliser les valeurs de signaux optiques pour déterminer la valeur de correction pour la concentration du matériau cible.

10. Procédé de mesure pour déterminer une concentration d'un matériau cible dans un spécimen, le procédé comprenant :
la réception du spécimen dans au moins deux récipients (45b), chaque récipient (45b) contenant un réactif configuré pour réagir avec le matériau cible dans le spécimen pour mesurer la concentration du matériau cible, dans lequel chaque réactif comprend en outre une concentration prédéterminée de matériau étalon interne contenant le matériau cible, dans lequel le matériau étalon interne est configuré pour libérer le matériau cible tout en étant dissous pendant un processus de mélange avec le spécimen et dans lequel les concentrations des matériaux étalons internes dans les réactifs des au moins deux récipients (45b) sont différentes :

la détection de caractéristiques optiques à partir d'une réaction entre le matériau cible dans le spécimen et les matériaux étalons internes avec les réactifs dans les au moins deux récipients (45b) et la fourniture de valeurs de signaux optiques ;
la dérivation d'une courbe de calibration mesurée à partir d'une étendue d'un changement dans la valeur de signal optique du matériau cible mesurée dans les au moins deux récipients (45b) ;
**caractérisé en ce que** le procédé comprend en outre :

l'obtention, à partir d'informations d'identification fournies sur la cartouche et/ou à partir d'informations sur la propriété du réactif stockées dans une mémoire de l'appareil, d'une courbe de calibration d'usine indiquant une étendue prédéterminée d'un changement dans la valeur de signal optique du matériau cible dans les au moins deux récipients ;
la détermination, sur la base d'une comparaison de la courbe de calibration mesurée avec la courbe de calibration d'usine, d'une valeur de correction pour le calcul de la concentration du matériau cible ; et
la détermination d'une concentration corrigée du matériau cible sur la base de la valeur de correction.

11. Procédé de la revendication 10,
dans lequel la détermination de la valeur de correction pour la concentration du matériau cible comprend :
le calcul d'une pente de changement dans une absorbance sur la base d'une absorbance du matériau cible mesurée à partir de l'un des au moins deux récipients (45b), d'une absorbance du matériau cible mesurée à partir de l'autre, et d'une différence de concentrations du matériau cible entre les au moins deux récipients (45b), la valeur de correction pour la concentration du matériau cible étant déterminée en utilisant la pente calculée du changement dans l'absorbance.

12. Procédé de la revendication 10,
dans lequel la détermination de la valeur de correction pour la concentration du matériau cible comprend :
le calcul d'une quantité de changement d'une absorbance sur la base d'une absorbance du matériau cible mesurée à partir de l'un des au moins deux récipients (45b), d'une absorbance du matériau cible mesurée à partir de l'autre, et d'une différence de concentrations du matériau cible entre les au moins deux récipients (45b), la valeur de correction pour la concentration du matériau cible étant déterminée en utilisant la quantité calculée de changement dans l'absorbance en fonction de la différence de concentration du matériau cible et en comparant le changement dans l'absorbance calculé avec un changement d'absorbance prédéterminé.

13. Procédé de la revendication 10,
dans lequel la détermination de la valeur de correction pour la concentration du matériau cible comprend :
le calcul d'une pente d'un changement dans une luminance sur la base d'une luminance du matériau cible mesurée à partir de l'un des au moins deux récipients (45b), d'une luminance du matériau cible mesurée à partir de l'autre, et d'une différence de concentrations du matériau cible entre les au moins deux récipients (45b), la valeur de correction pour la concentration du matériau cible étant déterminée en utilisant la pente calculée de changement dans la luminance.

14. Procédé de la revendication 10,
dans lequel la détermination de la valeur de correction pour la concentration du matériau cible comprend :
le calcul d'une pente de changement dans une fluorescence sur la base d'une fluorescence du matériau cible mesurée à partir de l'un des au moins deux récipients (45b), d'une fluorescence du matériau cible mesurée à partir de l'autre, et d'une différence de concentrations du matériau cible entre les au moins deux récipients (45b), la valeur de correction pour la concentration du matériau cible étant déterminée en utilisant la pente calculée de changement dans la fluorescence.

15. Procédé de la revendication 10, comprenant en outre les étapes de :

détermination d'au moins une région entre au moins deux concentrations de spécimen dans la courbe de calibration mesurée correspondant à une différence de concentrations du matériau cible entre les au moins deux récipients (45b) et d'une étendue d'un changement dans une valeur de signal optique du matériau cible pour chaque région, et

comparaison de l'étendue du changement dans la valeur de signal optique du matériau cible pour chaque région avec la courbe de calibration d'usine dérivée de l'étendue prédéterminée d'un changement dans la valeur de signal optique du matériau cible pour la région, pour déterminer une valeur de correction pour une concentration du matériau cible pour chaque région ; et/ou

création d'un tracé de changements de concentration en fonction d'une absorbance du matériau cible sur la base de la valeur de correction déterminée pour la concentration du matériau cible ; et/ou

détermination si les valeurs de signaux optiques du matériau cible mesurées à partir des au moins deux récipients (45b) se trouvent dans une plage de mesure prédéterminée, et sur la base de la détermination, détermination s'il faut utiliser les valeurs de signaux optiques pour déterminer la valeur de correction pour la concentration du matériau cible.

# FIG. 1

# FIG. 2

# FIG. 3

# FIG. 4

<u>40</u>

# FIG. 5

# FIG. 6

40

**FIG. 7**

# FIG. 8

1

**FIG. 9**

# FIG. 10A

# FIG. 10B

# FIG. 11A

# FIG. 11B

# FIG. 12

# FIG. 13

# FIG. 14A

## FIG. 14B

# FIG. 15

1410                1400

| < Home | Jordan | Show detail |
|---|---|---|

12/Jun/2012 09:50AM

| Cartridge : Q01 | k : 8.5 |
|---|---|
| | |

1420

# FIG. 16

1410                  1400

# FIG. 17

START

INJECT SPECIMEN TO AT LEAST TWO
CONTAINERS HAVING DIFFERENT
CONCENTRATIONS OF
INTERNAL STANDARD MATERIAL ~1700

MEASURE ABSORBANCE IN
AT LEAST TWO CONTAINERS ~1710

COMPARE EXTENT OF CHANGE IN ABSORBANCE
ACCORDING TO DIFFERENCE IN CONCENTRATION
OF TARGET MATERIAL BETWEEN CONTAINERS
WITH PREDETERMINED EXTENT OF CHANGE IN
ABSORBANCE TO DETERMINE CORRECTION VALUE
FOR CONCENTRATION OF TARGET MATERIAL ~1720

END

# FIG. 18

START

↓

CALCULATE MEASUREMENT VALUE OF TARGET MATERIAL IN AT LEAST TWO CONTAINERS WITH DIFFERENT CONCENTRATIONS OF INTERNAL STANDARD MATERIAL INJECTED THERETO —1800

↓

DETERMINE WHETHER CALCULATED MEASUREMENT VALUE IS WITHIN PREDETERMINED MEASUREMENT RANGE —1810

↓

SELECT MEASUREMENT VALUE TO BE USED IN DETERMINING CORRECTION VALUE BASED ON THE DETERMINATION —1820

↓

DETERMINE CORRECTION VALUE FOR CONCENTRATION OF TARGET MATERIAL USING SELECTED MEASUREMNT VALUE —1830

↓

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014058750 A1
- EP 0453036 A2
- US 2003157726 A1
- DE 3406223 A1
- US 2013203613 A1
- EP 2813839 A1
- EP 2618161 A1
- US 5462879 A